# EUROPEAN PATENT SPECIFICATION

(11) **EP 4 458 520 B1**
(45) Date of publication and mention of the grant of the patent: **04.02.2026**
(21) Application number: 24200563.5
(22) Date of filing: 03.12.2020
(51) Int. Cl.: A61H 3/00, A61B 5/11, A63B 23/04, B25J 9/00

(54) **METHOD AND DEVICE FOR PROVIDING RESISTANCE TO USER OF WEARABLE DEVICE**
VERFAHREN UND VORRICHTUNG ZUR BEREITSTELLUNG VON WIDERSTAND FÜR BENUTZER EINER WEARABLE-VORRICHTUNG
PROCÉDÉ ET DISPOSITIF POUR FOURNIR UNE RÉSISTANCE À UN UTILISATEUR D'UN DISPOSITIF PORTABLE

(30) Priority: 03.12.2019 KR 20190158819; 21.10.2020 KR 20200136562
(43) Date of publication of application: 06.11.2024
(62) Divisional of application: 20896890.9
(73) Proprietor: Samsung Electronics Co., Ltd., Gyeonggi-do, 16677 (KR)
(72) Inventor: Roh, Changhyun, Gyeonggi-do,16677 (KR)
(74) Representative: Gulde & Partner

(56) References cited:
- CN-A- 109 289 167
- US-A1- 2009 011 907
- US-A1- 2014 142 475

## Description

### 1. Technical Field

At least one example embodiment relates to a method and/or device for providing resistance to a user of a wearable device. For example, at least one example embodiment relates to a method and/or device for providing resistance to a user of a wearable device without providing energy to a motor of the wearable device.

### 2. Description of the Related Art

A change into aging societies has contributed to a growing number of people who experience inconvenience and pain from reduced muscular strength or joint problems due to aging. Thus, there is a growing interest in walking assist devices that enable elderly users or patients with reduced muscular strength or joint problems to walk with less effort.

CN 109289167 A, US 2014/142475 A1 relate to conventional devices for assisting movement of a user, without an exercise mode.

### SUMMARY

The invention is defined by the appended claims. According to one aspect, a method of controlling a motor driver circuit of a wearable device, a non-transitory computer-readable medium, and a werable device are provided as defined by the appended claims. The description that follows is subjected to this limitation. Any disclosure lying outside the scope of said claims is only intended for illustrative as well as comparative purposes.

Some example embodiments relate to a method of operating a wearable device to provide a resistance force to a user.

In some example embodiments, the method includes measuring, via a sensor, a first angle of a first joint of the user, determining a resistance level to apply to the first joint based on the first angle, determining a connection ratio between a connected time for which a motor driver circuit electrically connected to a motor of the wearable device is controlled to be a closed loop and a disconnected time for which the motor driver circuit is controlled to be an open loop, based on the resistance level, and controlling the motor through the motor driver circuit based on the connection ratio.

In some example embodiments, the motor driver circuit may include at least one switch configured to be controlled based on the connection ratio.

In some example embodiments, the connection ratio may be represented by a pulse width modulation (PWM).

In some example embodiments, the resistance level to be provided to the user may be adjusted based on the connection ratio such that as the connected time for which the motor driver circuit is controlled to be the closed loop increases, the resistance force may increase.

In some example embodiments, when the motor driver circuit is controlled to be the closed loop, the motor may operate as a generator with respect to an external force by the user.

In some example embodiments, when the motor operates as the generator, the method may further include charging a battery of the wearable device based on energy generated by the generator.

In some example embodiments, the method may further include receiving, from the user, an instruction to set an operation mode of the wearable device to an exercise mode.

In some example embodiments, when the exercise mode is set, the motor may not be provided with energy from the battery of the wearable device.

In some example embodiments, the method may further include receiving, from the user, an instruction to set an operation mode of the wearable device to an assistance mode, calculating an assistance torque value to apply to the first joint based on the first angle in response to the assistance mode being received, and providing an assistance force to the user by controlling the motor based on the assistance torque value.

Some example embodiments relate to a wearable device configured to provide a resistance force to a user.

In some example embodiments, the wearable device includes a memory configured to store a program that includes instructions to provide the resistance force to the user, a sensor configured to measure a first angle of a first joint of the user, a motor driver circuit, a motor electrically connected to the motor driver circuit, and a processor configured to execute the program to measure, via the sensor, the first angle of the first joint of the user, determine a resistance level to apply to the first joint based on the first angle, determine a connection ratio between a connected time for which the motor driver circuit is controlled to be a closed loop and a disconnected time for which the motor driver circuit is controlled to be an open loop based on the resistance level, and control the motor through the motor driver circuit based on the connection ratio.

In some example embodiments, the motor driver circuit may include at least one switch configured to be controlled based on the connection ratio.

In some example embodiments, the connection ratio may be represented by a PWM, and the resistance force to be provided to the user may be adjusted based on the connection ratio such that, as the connected time for which the motor driver circuit is controlled to be the closed loop increases, the resistance force may increase.

In some example embodiments, when the motor driver circuit is controlled to be the closed loop, the motor may operate as a generator with respect to an external force by the user.

In some example embodiments, when the motor operates as the generator, the processor is further configured to charge a battery of the wearable device based on energy generated by the generator.

In some example embodiments, the processor is further configured to receive, from the user, an instruction to set an operation mode of the wearable device to an exercise mode, and determine the resistance level to apply to the first joint based on the exercise mode and the first angle.

In some example embodiments, when the exercise mode is set, the motor may not be provided with energy from the battery by the wearable device.

In some example embodiments, the processor is further configured to, receive, from the user, an instruction to set an operation mode of the wearable device to an assistance mode, calculate an assistance torque value to apply to the first joint based on the first angle in response to the assistance mode being received, and provide an assistance force to the user by controlling the motor based on the assistance torque value.

Additional aspects of example embodiments will be set forth in part in the description which follows and, in part, will be apparent from the description, or may be learned by practice of the disclosure.

### BRIEF DESCRIPTION OF THE DRAWINGS

These and/or other aspects will become apparent and more readily appreciated from the following description of example embodiments, taken in conjunction with the accompanying drawings of which:
FIGS. 1A through 1D are diagrams illustrating an example of a wearable device according to at least one example embodiment;
FIG. 2 is a diagram illustrating an example of a wearable device communicating with an electronic device according to at least one example embodiment;
FIG. 3 is a diagram illustrating an example of a gait state according to at least one example embodiment;
FIG. 4 is a diagram illustrating an example of a transition in gait state according to at least one example embodiment;
FIG. 5 is a diagram illustrating an example of a trajectory of an ankle joint angle with respect to a gait cycle according to at least one example embodiment;
FIG. 6 is a diagram illustrating an example of a trajectory of an ankle torque with respect to a gait cycle according to at least one example embodiment;
FIGS. 7 through 10 are diagrams illustrating an example of a motor driver circuit of a wearable device according to at least one example embodiment;
FIG. 11 is a flowchart illustrating an example of a method of providing a resistance force according to at least one example embodiment;
FIG. 12 is a diagram illustrating an example of a resistance force profile output from a user terminal according to at least one example embodiment;
FIG. 13 is a flowchart illustrating another example of a method of providing a resistance force according to at least one example embodiment;
FIG. 14 is a diagram illustrating an example of a motor driver circuit of an open loop according to at least one example embodiment;
FIGS. 15 and 16 are diagrams illustrating examples of a motor driver circuit of a closed loop according to at least one example embodiment;
FIG. 17 is a diagram illustrating an example of a motor driver circuit of a closed loop according to at least one example embodiment;
FIG. 18 is a diagram illustrating an example of a motor driver circuit of a closed loop including a braking resistor according to at least one example embodiment;
FIG. 19 is a diagram illustrating an example of a motor driver circuit of a closed loop including a resistor according to at least one example embodiment;
FIG. 20 is a diagram illustrating an example of a motor driver circuit of a closed loop including a brushless direct current (BLDC) motor according to at least one example embodiment;
FIG. 21 is a diagram illustrating an example of a driver of a wearable device according to at least one example embodiment; and
FIGS. 22 through 24 are diagrams illustrating an example of a whole body-type wearable device according to at least one example embodiment.

### DETAILED DESCRIPTION

Hereinafter, some example embodiments will be described in detail with reference to the accompanying drawings. Regarding the reference numerals assigned to the elements in the drawings, it should be noted that the same elements will be designated by the same reference numerals, wherever possible, even though they are shown in different drawings. Also, in the description of embodiments, detailed description of well-known related structures or functions will be omitted when it is deemed that such description will cause ambiguous interpretation of the present disclosure.

It should be understood, however, that there is no intent to limit this disclosure to the particular example embodiments disclosed. On the contrary, example embodiments are to cover all modifications, equivalents, and alternatives falling within the scope of the example embodiments. Like numbers refer to like elements throughout the description of the figures.

In addition, terms such as first, second, A, B, (a), (b), and the like may be used herein to describe components. Each of these terminologies is not used to define an essence, order or sequence of a corresponding component but used merely to distinguish the corresponding component from other component(s). It should be noted that if it is described in the specification that one component is "connected," "coupled," or "joined" to another component, a third component may be "connected," "coupled," and "joined" between the first and second components, although the first component may be directly connected, coupled or joined to the second component.

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Unless otherwise defined, all terms, including technical and scientific terms, used herein have the same meaning as commonly understood by one of ordinary skill in the art to which the disclosure of this application pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Also, in the description of example embodiments, detailed description of structures or functions that are thereby known after an understanding of the disclosure of the present application will be omitted when it is deemed that such description will cause ambiguous interpretation of the example embodiments.

Various example embodiments will now be described more fully with reference to the accompanying drawings in which some example embodiments are shown. In the drawings, the thicknesses of layers and regions are exaggerated for clarity.

Hereinafter, examples will be described in detail with reference to the accompanying drawings, and like reference numerals in the drawings refer to like elements throughout.

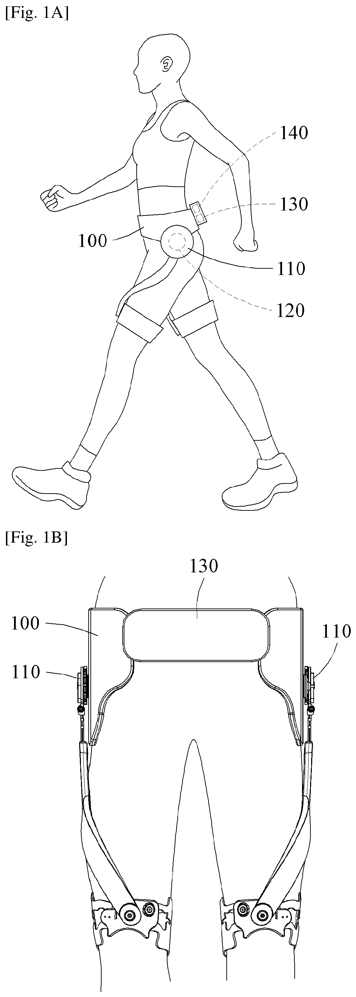

FIGS. 1A through 1D are diagrams illustrating an example of a wearable device according to at least one example embodiment.

Referring to FIGS. 1A through 1C, a wearable device 100 may be worn on a user and configured to assist the user in walking more readily. For example, the wearable device 100 may be a device that assists the user in walking. The wearable device 100 may also be an exercise device that provides an exercise function by providing the user with a resistance force to assist the user in doing an exercise. The resistance force to be provided to the user may not be a force that is actively applied to the user, for example, a force output by a device such as a motor, but a force that hinders a movement of the user, for example, a force acting in a direction opposite to a direction in which the user moves. That is, the resistance force may also be referred to as an exercise load.

Although FIGS. 1A and 1B illustrate an example of a hip-type wearable device, a type of a wearable device is not limited to the illustrated hip type, and the wearable device may be provided in a type that supports a whole lower body, supports a portion of the lower body, for example, a portion of the lower body up to a knee and a portion of the lower body up to an ankle, or supports a whole body.

Although example embodiments to be described hereinafter with reference to FIGS. 1A and 1B are applicable to a hip-type wearable device, for example, the wearable device 100, the example embodiments are not limited to the hip-type wearable device, but applicable to all types of wearable devices.

Referring to FIGS. 1A through 1D, the wearable device 100 includes a driver 110, a sensor 120, an inertial measurement unit (IMU) 130, a controller 140, and a battery 150.

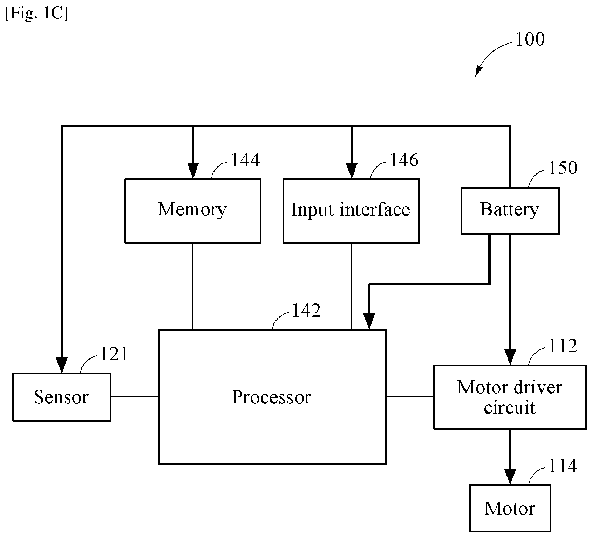

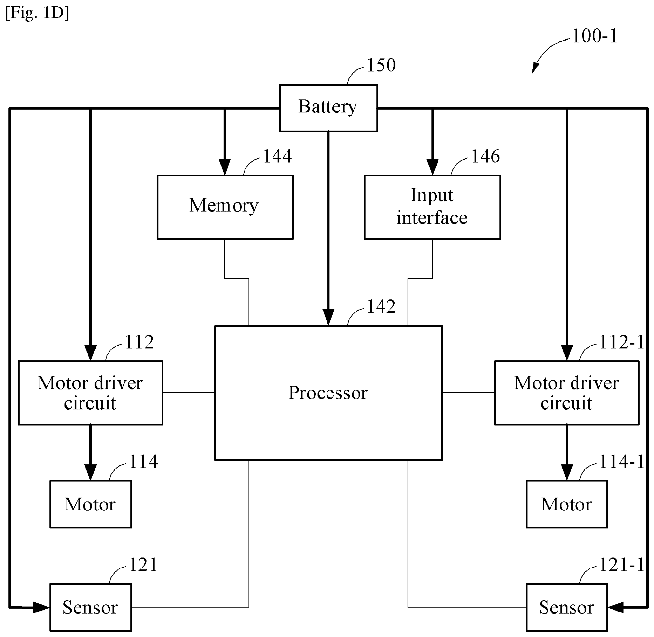

The driver 110 includes a motor 114 and a motor driver circuit 112 configured to drive the motor 114. The sensor 120 includes at least one sensor 121. The controller 140 includes a processor 142, a memory 144, and an input interface 146. Although a single sensor 121, a single motor driver circuit 112, and a single motor 114 are illustrated in FIG. 1C, examples are not limited thereto. For another example, a wearable device 100-1 may include a plurality of sensors 121 and 121-1, a plurality of motor driver circuits 112 and 112-1, and a plurality of motors 114 and 114-1, as illustrated in FIG. 1D. In addition, according to implementation, the wearable device 100 may include a plurality of processors. The number of motor driver circuits, the number of motors, or the number of processors may vary according to a body part on which the wearable device 100 is to be worn.

The following descriptions of the sensor 121, the motor driver circuit 112, and the motor 114 are also applicable to the sensor 121-1, the motor driver circuit 112-1, and the motor 114-1 illustrated in FIG. 1D.

The driver 110 may drive hip joints of the user wearing the wearable device 100. For example, the driver 110 may be disposed in a portion of a right hip of the user and/or a portion of a left hip of the user. The driver 110 may be additionally disposed in a portion of knees of the user and a portion of ankles of the user. The driver 110 includes the motor 114 configured to generate a rotational torque and the motor driver circuit 112 configured to drive the motor 114.

The sensor 120 may measure a hip joint angle of the user when the user walks. Here, information associated with the hip joint angle sensed by the sensor 120 may include an angle of a right hip joint, an angle of a left hip joint, a difference between the angle of the right hip joint and the angle of the left hip joint, and/or a hip joint movement direction. For example, the sensor 121 may be disposed in the driver 110. Based on a position of the sensor 121, the sensor 120 may additionally measure a knee angle of the user and an ankle angle of the user.

For example, the sensor 120 may include a potentiometer. The potentiometer may sense an R-axis joint angle and an L-axis joint angle, and an R-axis joint angular velocity and an L-axis joint angular velocity, based on a walking motion of the user.

The IMU 130 may measure acceleration information and pose information when the user walks. For example, the IMU 130 may sense an X-axis acceleration, a Y-axis acceleration, and a Z-axis acceleration, and an X-axis angular velocity, a Y-axis angular velocity, and a Z-axis angular velocity, based on a walking motion of the user.

The wearable device 100 may detect a point at which a foot of the user lands based on the acceleration information measured by the IMU 130.

In addition, a pressure sensor (not shown) may be disposed on a sole of a foot of the user and detect a point in time at which the foot of the user lands.

In addition to the sensor 120 and the IMU 130, the wearable device 100 may include other sensors configured to sense a change in a quantity of motion of the user or a change in biosignal based on a walking motion of the user. The sensors may include an electromyogram (EMG) sensor, for example.

According an example embodiment, the processor 142 of the controller 140 may control the driver 110 to provide a resistance force to the user. The driver 110 may provide the resistance force to the user through back-drivability of the motor 114 without outputting a torque to the user. Here, back-drivability of the motor 114 may indicate reactivity of a rotation axis of the motor 114 in response to an external force. For example, high back-drivability of the motor 114 may indicate readily responding to an external force applied to the rotation axis of the motor 114, that is, the rotation axis of the motor 114 rotates readily. For example, even though the same external force is applied to the rotation axis of the motor 114, a degree of a rotation of the rotation axis of the motor 114 may directly vary depending on a level of back-drivability.

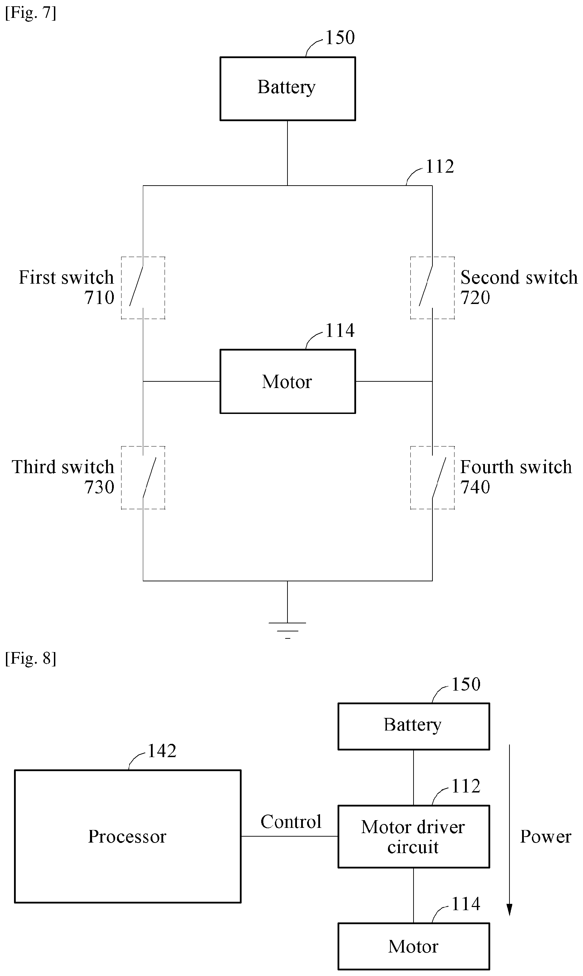

A method of providing a resistance force to a user will be described in detail hereinafter with reference to FIGS. 7 through 21.

According another example embodiment, the processor 142 of the controller 140 may control the driver 110 to output a torque, for example, an assistance torque, to assist the user in walking. For example, the driver 110 may be provided as two drivers for the right hip and the left hip, respectively, in the wearable device 100 of the hip type, and the controller 140 may output a control signal for controlling the driver 110 such that the torque is generated.

The driver 110 may generate the torque based on the control signal output by the controller 140. Here, a torque value used to generate the torque may be set externally or set by the controller 140. For example, to indicate a magnitude of the torque value, the controller 140 may use a magnitude of a current with respect to a signal transmitted to the driver 110. That is, as the magnitude of the current received by the driver 110 increases, the torque value may increase.

The battery 150 may provide power to components of the wearable device 100. For example, there may be a circuit, for example, a power management integrated circuit (PMIC) that is configured to convert power of the battery 150 to an operating voltage of each of the components of the wearable device 100 and then provide the power to the components. In addition, the battery 150 may provide or not provide power to the motor 114 based on an operation mode of the wearable device 100. That is, the battery 150 may provide power to the motor 114 in an assistance mode, and not provide power to the motor 114 in an exercise mode. Thus, less power may be consumed in the battery 150 in the exercise mode, and thus an available time for using the wearable device 100 may increase.

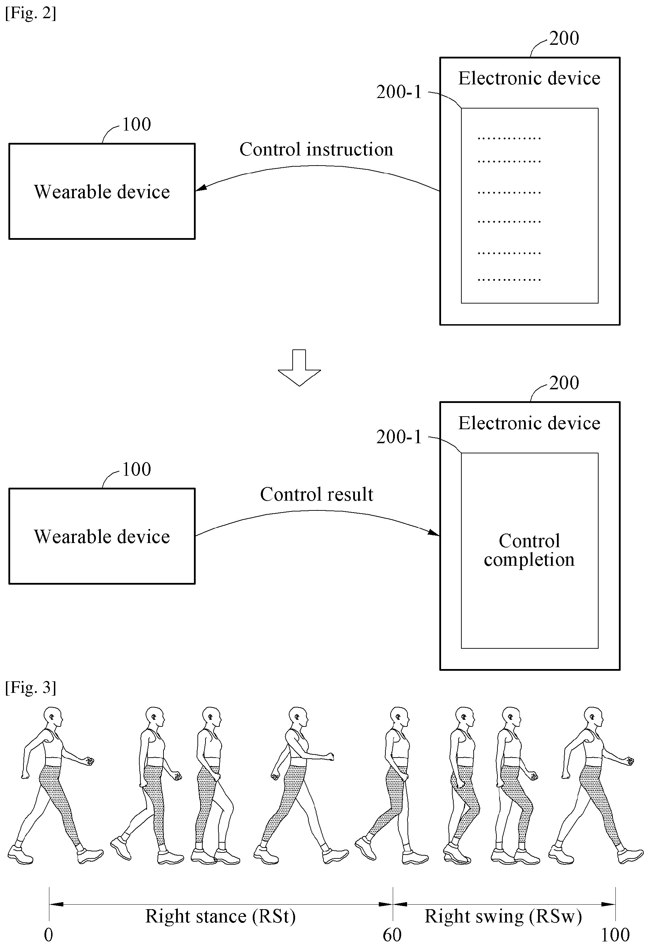

FIG. 2 is a diagram illustrating an example of a wearable device communicating with an electronic device according to at least one example embodiment.

Referring to FIG. 2, the wearable device 100 may communicate with an electronic device 200. The electronic device 200 may include, for example, a smartphone, a tablet personal computer (PC), a smart watch, eyeglasses, and the like, but examples are not limited thereto. For example, the electronic device 200 may be an electronic device associated with a user of the wearable device 100. For another example, the user may do exercise together with a trainer with the wearable device 100 on. In such a case, the electronic device 200 may be an electronic device associated with the trainer.

According to implementation, the wearable device 100 and the electronic device 200 may communicate with each other via a server (not shown) using a short-range wireless communication method or a cellular mobile communication method.

The electronic device 200 may display, on a display 200-1, a user interface (UI) for controlling an operation of the wearable device 100. For example, the UI may include at least one softkey that enables the user to control the wearable device 100.

The user or the trainer may input a control instruction for controlling an operation of the wearable device 100 through the UI on the display 200-1 of the electronic device 200. The electronic device 200 may transmit the control instruction to the wearable device 100. The wearable device 100 may operate according to the received control instruction, and transmit a control result to the electronic device 200. The electronic device 200 may display a message indicating control completion on the display 200-1 of the electronic device 200.

FIG. 3 is a diagram illustrating an example of a gait state according to at least one example embodiment.

Referring to FIG. 3, a gait state or a gait phase of one of legs of a user of a wearable device may be defined (or, alternatively, predefined). For example, the gait state may include a stance and a swing. The swing indicates a state in which a foot is away from the ground. A gait state of a left leg of the user may include a left stance (LSt) and a left swing (LSw), and a gait state of a right leg of the user may include a right stance (RSt) and a right swing (RSw).

In a finite-state machine (FSM), a gait cycle of such gait states or phases may be mapped in advance. For example, 0% of the gait cycle may be mapped at a point in time at which a stance starts, 60% of the gait cycle may be mapped at a point in time at which a swing starts, and 100% of the gait cycle may be mapped at a point in time immediately before a next stance starts.

According to an example embodiment, the stance and the swing may be more elaborately classified into a plurality of states. The stance may be classified into, for example, an initial contact, a weight bearing, a middle stance, a terminal stance, and a pre-swing. In addition, the swing may be classified into, for example, an initial swing, a middle swing, and a terminal swing. However, the classification of the stance and the swing is not limited to the example classification described in the foregoing, and the stance and the swing may be differently classified according to examples.

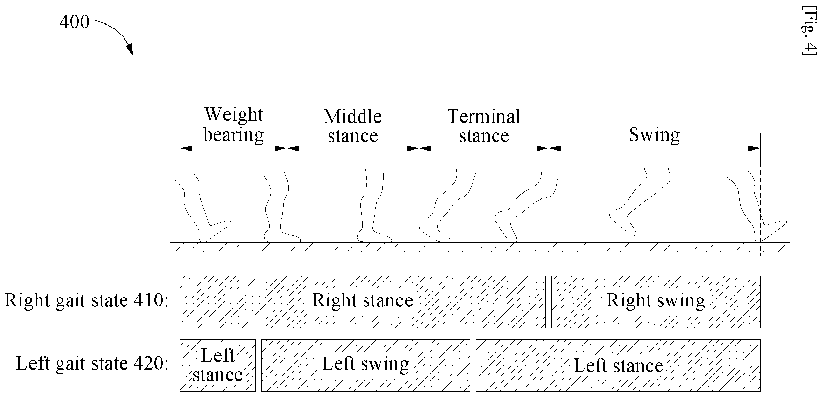

FIG. 4 is a diagram illustrating an example of a transition in gait state according to at least one example embodiment.

Referring to FIG. 4, according to a general gait mechanism, a gait state of each of both legs may include a stance and a swing, and the stance and the swing may occur alternately for walking.

A gait state 410 of a right leg according to a change 400 in the right leg by walking may include a right stance and a right swing. Here, the stance may include a weight bearing, a middle stance, and a terminal stance. However, examples are not limited to the illustrated example. A gait state 420 of a left leg according to a change (not shown) in the left leg with respect to the change 400 in the right leg may include a left stance and a left swing.

When muscular strength of an ankle of a user weakens due to aging or a disease, the user may experience inconvenience when walking. For example, when a leg starts swinging, a tip of a foot of the leg needs to be raised, but if not the swinging leg may hit the ground. That is, in such a case of a foot drop, there may be a risk of a fall. To prevent such a risk, an angle of the ankle may need to be adjusted based on a progression of a gait state or phase or a change in the gait state or phase. Thus, a wearable device may be provided to those who may have difficulty in adjusting an angle of an ankle themselves due to reduced muscular strength of the ankle. The wearable device may be worn around an ankle of a user and output an assistance torque based on a value sensed in association with walking or gait of the user. The assistance torque may be used to adjust an angle of the ankle of the user.

Although the example embodiment described above relates to assisting or supporting an ankle, the example embodiment may also be similarly and substantially applied to assisting or supporting a hip joint or a knee.

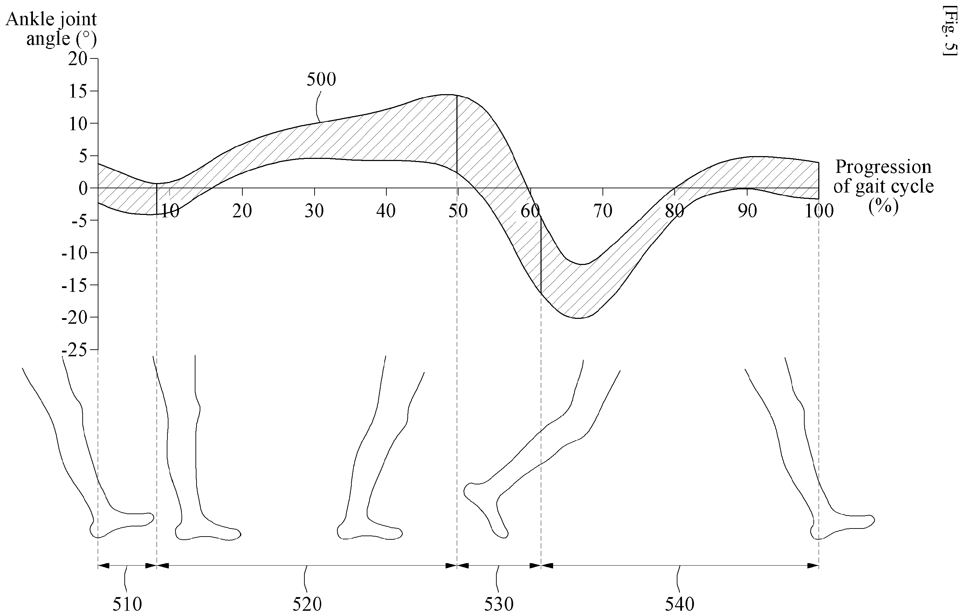

FIG. 5 is a diagram illustrating an example of a trajectory of an ankle joint angle with respect to a gait cycle according to at least one example embodiment.

Referring to FIG. 5, when a user walks according to a general gait mechanism, an ankle joint angle of the user may change as shown in a trajectory 500. Although an ankle joint angle may change even in the same gait state based on a stride and a walking speed, trajectories of an ankle joint angle with respect to one gait cycle may develop in a similar pattern. The trajectory 500 may have an illustrated range of changes in a progression of a gait cycle. The range of changes may include weight bearing 510, middle stance 520, terminal stance 530 and swing 540.

However, the illustrated trajectory 500 may not be shown in an ankle joint angle of a leg of a patient who may experience inconvenience with the leg. Here, when the ankle joint angle of the leg from which the patient may experience inconvenience is adjusted such that the ankle joint angle of the leg has the trajectory 500, a gait mechanism of the patient may be improved.

Although a trajectory of an ankle joint angle has been described above with reference to FIG. 5, the foregoing description may be similarly and substantially applied to a hip joint ankle and a knee joint.

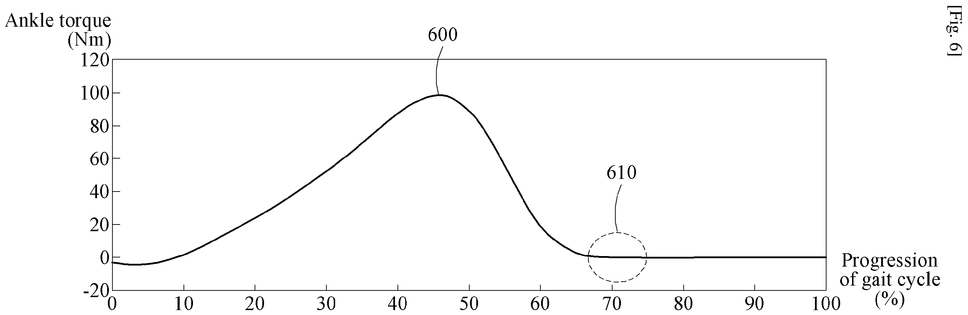

FIG. 6 is a diagram illustrating an example of a trajectory of an ankle torque with respect to a gait cycle according to at least one example embodiment.

Referring to FIG. 6, what is to be described hereinafter with reference to FIG. 6 may be applied to a case in which the wearable device 100 operates in an assistance mode in which the wearable device 100 assists a user wearing the wearable device 100 in walking. A case in which the wearable device 100 operates in an exercise mode will be described in detail with reference to FIGS. 7 through 21.

When a user walks according to a general gait mechanism, an ankle torque to be output by an ankle joint of the user may change as shown in a trajectory 600. A positive value of the ankle torque may increase an ankle joint angle, for example, enabling plantar flexion. A negative value of the ankle torque may decrease the ankle joint angle, for example, enabling dorsiflexion.

According to an example embodiment, a first portion 610 of the trajectory 600 of the ankle torque that corresponds to a segment after a push-off occurs may be an assistance torque value for the dorsiflexion to prevent a foot drop. The assistance torque value for the dorsiflexion may be a negative value.

A patient who may experience inconvenience with his/her leg may not generate a sufficient assistance torque himself/herself, and thus the patient may wear a wearable device on the leg from which the patient experiences inconvenience to receive the assistance torque. The wearable device may output the assistance torque through a driver and adjust an ankle angle. Here, the assistance torque for adjusting the ankle angle may need to be output at a desirable timing so that the user may not experience inconvenience. For example, a relatively high assistance torque for increasing the ankle angle may need to be provided at a time at which the leg performs a push-off. For example, the timing may be determined by directly determining a gait state of the leg from which the patient experiences inconvenience. For another example, the timing may be determined by indirectly determining a gait state of the leg based on a gait state of the other normal leg from which the patient does not experience inconvenience.

Hereinafter, a structure of the motor driver circuit 112 included in the driver 110 that enables the wearable device 100 to operate in an exercise mode that does not require use of the battery 150 will be described in detail with reference to FIGS. 7 through 10.

FIGS. 7 through 10 are diagrams illustrating an example of a motor driver circuit of a wearable device according to at least one example embodiment.

Referring to FIG. 7, the motor driver circuit 112 is an H-bridge circuit and includes a plurality of switches, for example, a first switch 710, a second switch 720, a third switch 730, and a fourth switch 740. The motor driver circuit 112 is connected to the motor 114.

For example, when the first switch 710 and the fourth switch 740 are closed and the second switch 720 and the third switch 730 are open under the control of the processor 142, a closed loop including the battery 150 may be formed. Thus, power may be provided to the motor 114 from the battery 150. In such an example, the motor 114 may rotate in a first direction.

For another example, when the second switch 720 and the third switch 730 are closed and the first switch 710 and the fourth switch 740 are open under the control of the processor 142, a closed loop including the battery 150 may be formed. Thus, power may be provided to the motor 114 from the battery 150. In such an example, the motor 114 may rotate in a second direction which is opposite to the first direction.

Referring to FIG. 8, the motor driver circuit 112 may be controlled such that a closed loop including the battery 150 is formed. The battery 150 may provide power to the motor 114. The motor 114 may rotate in a direction corresponding to a direction of a current. For example, when the wearable device 100 operates in an assistance mode, the processor 142 may control the motor driver circuit 112 such that the closed loop including the battery 150 is formed.

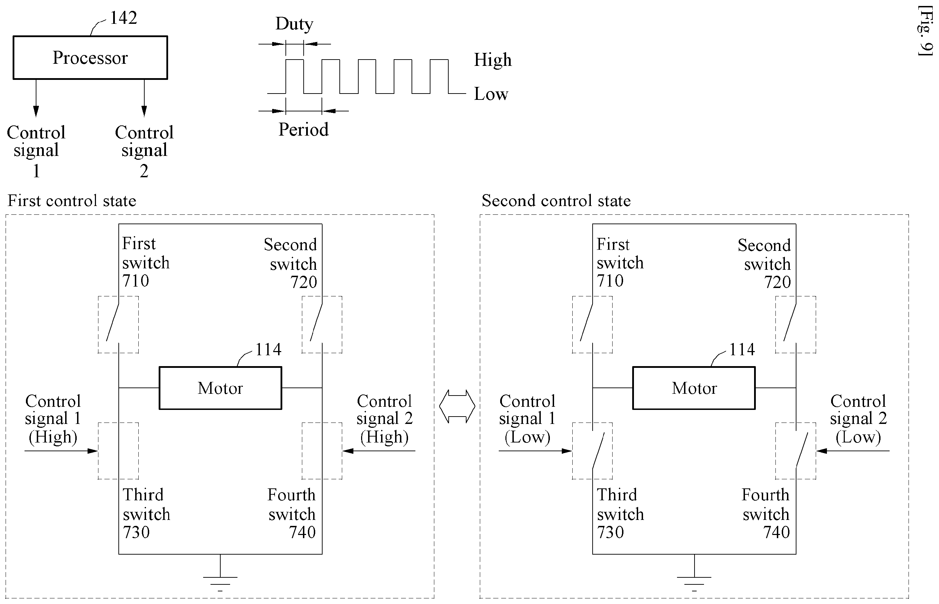

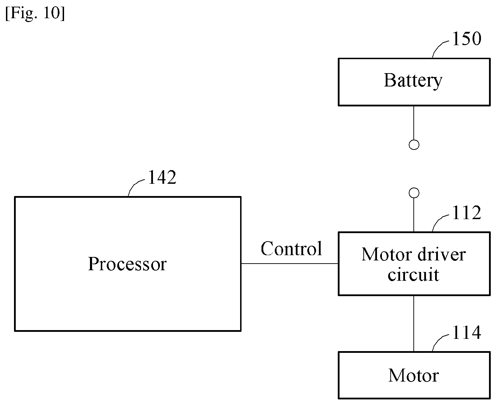

Dissimilar to what is described above with reference to FIGS. 7 and 8, the motor driver circuit 112 may also be controlled such that the battery 150 is excluded. Such an example will be described hereinafter with reference to FIGS. 9 and 10. The example of controlling the motor driver circuit 112 as described hereinafter with reference to FIGS. 9 and 10 may be used when an operation mode of the wearable device 100 is an exercise mode.

Referring to FIG. 9, the processor 142 may block an electrical connection between the battery 150 and the motor 114 by opening the first switch 710 and the second switch 720. In an exercise mode, the first switch 710 and the second switch 720 may remain opened. In the exercise mode, a lower driver circuit including the third switch 730 and the fourth switch 740 connected to the motor 114 may only be controlled.

The processor 142 may apply control signal 1 to the third switch 730 and apply control signal 2 to the fourth switch 740 such that a control state of the motor driver circuit 112 changes between a first control state and a second control state. Here, control signal 1 and control signal 2 may be in a form of a PWM in which a high value and a low value repeatedly alternate and have a duty ratio. The duty ratio may indicate a connection ratio. In a case in which one period is T and a time for which a high value is maintained in one period T is t_{H}, the duty ratio is t_{H}/T. Although the outputting of control signals 1 and 2 by the processor 142 is described herein, examples are not limited thereto. For example, the processor 142 may output a single control signal, and the output control signal may be divided by a separate circuit. Control signals obtained through the dividing may be applied to the third switch 730 and the fourth switch 740, respectively.

For example, when control signals 1 and 2 are high values, a (+) terminal and a (-) terminal of the motor 114 may be connected to be in an equipotential state. That is, in the first control state, the (+) terminal and the (-) terminal of the motor 114 may be electrically connected to have the same potential or voltage.

In the first control state, the motor 114 may form a closed loop with the ground without an electrical connection with the battery 150. Thus, the first control state may also be referred to as a closed loop state of the motor 114 free of the electrical connection with the battery 150.

When a user moves in the first control state, the motor 114 disposed around a corresponding joint of the user may rotate by the movement of the joint of the user. By such a rotation, an electromotive force or a potential difference may occur in the motor 114. The terminals of the motor 114 in the first control state may be in the equipotential state, and thus a rotation resistance may be generated in the motor 114 to reduce the generated electromotive force. This rotation resistance may be provided to the user as a resistance force.

For example, when control signals 1 and 2 are low values, the (+) terminal and the (-) terminal of the motor 114 may be electrically opened. In the second control state, there is no electrical connection to the motor 114. Thus, the second control state may also be referred to as an open loop state of the motor 114.

When a user moves in the second control state, the motor 114 may rotate by the movement of the user. In the second control state, the (+) terminal and the (-) terminal of the motor 114 may be electrically opened, and thus an electromotive force may not occur in the motor 114 and the resistance force may not be output. That is, back-drivability of the motor 114 may increase, and a frictional force by a gear ratio may only be felt or experienced by the user as the resistance force.

Based on the high values and the low values of control signals 1 and 2 may repeat according to a PWM signal, and thus the control sate of the motor driver circuit 112 may be switched repeatedly between the first control state and the second control state.

The processor 142 may adjust a magnitude of the resistance force by controlling a duty ratio of each of control signals 1 and 2. For example, when a time for which a high value is maintained increases in a period of each of control signals 1 and 2 (that is, a time for which a low value is maintained decreases), a ratio of the motor 114 operating in the first control state in a period of each of control signals 1 and 2 may increase compared to a ratio of the motor 114 operating in the second control state in a period of each of control signals 1 and 2. Thus, an intensity of the resistance force to be output to the user may increase. In contrast, when a time for which a high value is maintained decreases in a period of each of control signals 1 and 2 (that is, a time for which a low value is maintained increase), a ratio of the motor 114 operating in the second control state in a period of each of control signals 1 and 2 may increase compared to a ratio of the motor 114 operating in the first control state in a period of each of control signals 1 and 2. Thus, an intensity of the resistance force to be output to the user may decrease.

In the exercise mode, the wearable device 100 may output a resistance force without providing power of the battery 150 to the motor 114, and it is thus possible to consume less power of the battery 150 and increase an available time for using the wearable device 100. When power of the battery 150 is provided to the motor 114, the motor 114 may malfunction. However, in the exercise mode, the power of the battery 150 may not be provided to the motor 114, and thus a potential malfunction of the motor 114 may be prevented and the safety of the wearable device 100 may be improved further.

Referring to FIG. 10, the motor driver circuit 112 may be controlled not to include the battery 150. The battery 150 may be electrically disconnected from the motor 114. Based on a connection state of the motor driver circuit 112, the motor 114 may generate an electromotive force (in a case in which the connection state corresponds to a closed loop) by an external force or may not generate the electromotive force (in a case in which the connection state corresponds to an open loop).

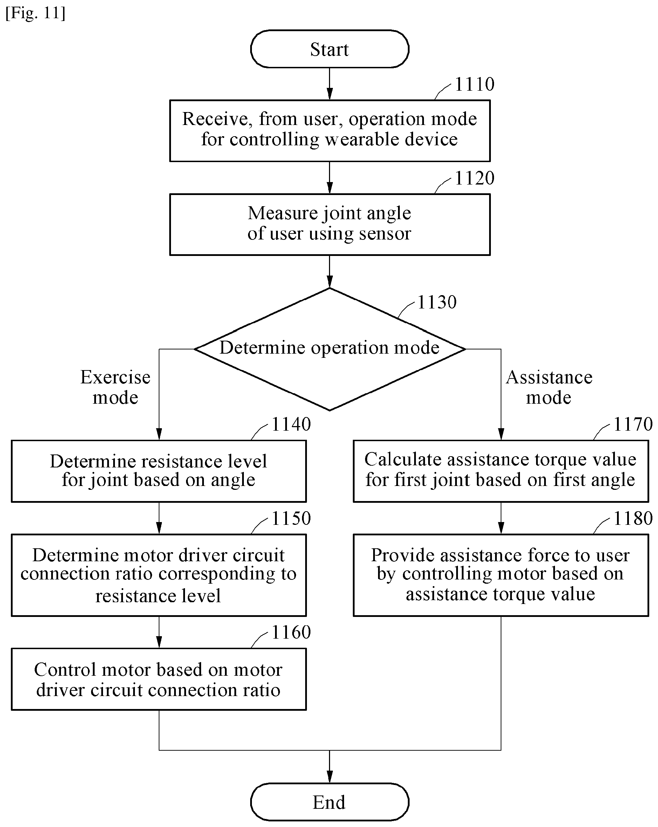

FIG. 11 is a flowchart illustrating an example of a method of providing a resistance force according to at least one example embodiment.

Operations 1110 through 1180 to be described hereinafter with reference to FIG. 11 may be performed by the wearable device 100. Although the wearable device 100 is described above as being worn on a lower body of a user, examples of the wearable device 100 are not limited thereto. For example, the wearable device 100 may be worn on an upper body of a user. For another example, the wearable device 100 may be worn throughout a whole body of a user.

Referring to FIG. 11, in operation 1110, the wearable device 100 receives, from a user, an operation mode that controls the wearable device 100. The operation mode may include an exercise mode and an assistance mode, and the received operation mode may be the exercise mode or the assistance mode.

For example, the user may transmit the operation mode to the wearable device 100 through a user terminal connected to the wearable device 100 through a wireless network. The wearable device 100 may receive the operation mode through a communication module. The wireless network may include, for example, a cellular network, a Bluetooth network, a WiFi network, and the like, but examples are not limited thereto.

For another example, the user may input the operation mode through the input interface 146 of the wearable device 100. The input interface 146 may include, for example, a physical button for receiving an input of the user, a software button formed based on a touch panel, a display and an indicator for outputting a state of the wearable device 100, and the like. The indicator may be, for example, a light-emitting diode (LED), but examples are not limited thereto.

A plurality of dynamic modes that control the wearable device 100 may be provided in advance in the wearable device 100.

For example, the wearable device 100 may control an operation of the wearable device 100 through a control algorithm. The control algorithm may be an algorithm that outputs a control value corresponding to an input including, for example, an input from the user and an input from the sensor 120, the IMU 130, and the like. For example, the control algorithm may operate based on a control table indicating an output value corresponding to input values.

For another example, the wearable device 100 may control an operation of the wearable device 100 based on a neural network corresponding to each operation mode rather than through a control algorithm. The neural network may be an artificial neural network, and be trained in advance through machine learning. The neural network may be, for example, a convolutional neural network (CNN), a recurrent neural network (RNN), a deep neural network (DNN), and a combination thereof, but examples are not limited thereto.

In response to an input being received, the neural network may output a result for the input. For example, when an angle of a joint is input, a neural network trained with the assistance mode may determine a gait state corresponding to the angle and calculate an assistance torque value for the joint. For another example, when an angle of a joint is input, a neural network trained with the exercise mode may determine a gait state corresponding to the angle and calculate and output a resistance level for the joint. In this example, the resistance level to be calculated may vary based on an exercise level set by the user. For example, when a higher exercise level is set, a higher resistance level may be calculated.

In operation 1120, the wearable device 100 measures an angle of a joint of the user using the sensor 121. The sensor 121 may be an angle sensor, and measure an angle of at least one of a hip joint, a knee joint, or an ankle joint. Alternatively, respective angles of a plurality of joints may be measured. The angles of the joints that are simultaneously measured may form a joint angle set with respect to a certain time. The joint angle set may be used to determine a level of a gait cycle of the user. For example, a leg pose of the user may be determined based on a hip joint angle, a knee joint angle, and an ankle joint angle. In addition, the joint angle set may additionally include an angular acceleration calculated based on a variation in the same joint angle.

Although the hip joint, the knee joint, and the ankle joint are described herein as examples, the foregoing description may also be applied to other joints including, for example, a shoulder joint, an elbow joint, and a wrist joint.

Although operation 1120 is illustrated as being performed between operations 1110 and 1130, operation 1120 may be performed continuously as long as power is provided to the sensor 121. The sensor 121 may generate data including, for example, a joint angle, on a preset period. That is, the joint angle may be measured successively and continuously.

In operation 1130, the processor 142 of the wearable device 100 determines whether the operation mode is the exercise mode or the assistance mode. When the operation mode is the exercise mode, a resistance force may be provided to the user. When the operation mode is the assistance mode, an assistance force may be provided to the user. To provide the resistance force to the user, operations 1140 through 1160 may be performed. To provide the assistance force to the user, operations 1170 and 1180 may be performed.

Although it is described that the operation mode is determined in operation 1130, whether the operation mode is the assistance mode may be determined in operation 1130 and then whether the operation mode is the exercise mode may be determined when the operation mode is determined not to be the assistance mode. Alternatively, whether the operation mode is the exercise mode may be determined in operation 1130 and then whether the operation mode is the assistance mode may be determined when the operation mode is determined not to be the exercise mode.

According to an example embodiment, when the user walks, the exercise mode may be applied in the entire gait state, or selectively applied in a certain gait state. For example, the exercise mode may be applied only in a swing state between a stance state and the swing state as the user chooses. Here, to determine a current gait state of the user, the angle of the joint of the user may be used.

In operation 1140, when the operation mode is the exercise mode, the processor 142 of the wearable device 100 determines the resistance level for the joint based on the measured angle of the joint. For example, through the control algorithm, a resistance level for an input of a joint angle may be determined. For another example, a resistance level may be output by inputting a joint angle to a neural network determined based on the operation mode.

The resistance level refers to a level or magnitude of a resistance force the user may feel or experience. For example, when the user desires to receive the same resistance force for an entire gait motion (or a running motion), the same resistance level may be determined for the entire gait motion. For another example, when the user desires to receive a different resistance force for a certain state or phase of a gait mechanism (e.g., a swing state), a level of a gait cycle of the user may be determined based on a joint angle (e.g., a joint angle set) and a resistance level corresponding to the determined level of the gait cycle may be determined. The level of the gait cycle may change in real time, and thus the resistance level to be determined may change in real time.

According to an example embodiment, a resistance force profile generated in advance may indicate a trajectory of a resistance level with respect to an entire gait cycle. Based on the resistance force profile with respect to the entire gait cycle, a resistance level corresponding to a level of a current gait cycle may be determined.

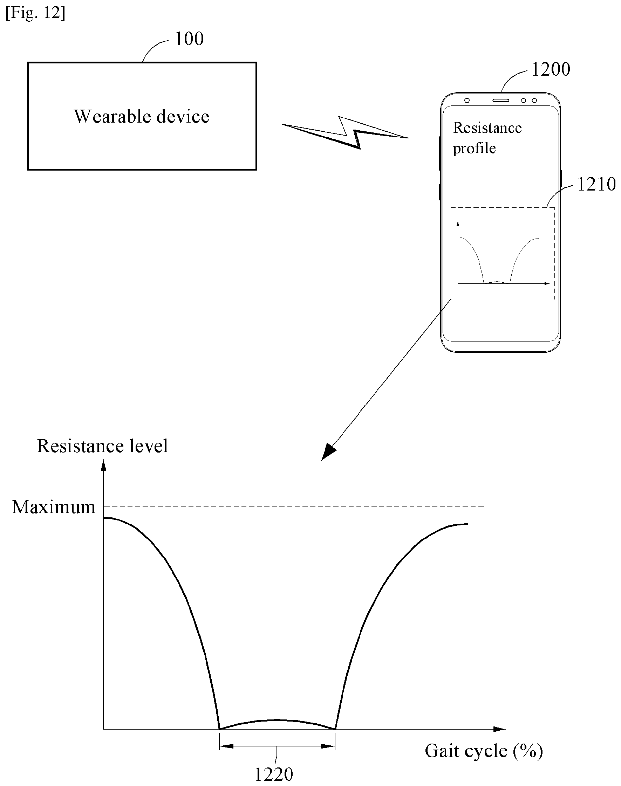

The resistance profile may be adjusted by the user. For example, the user may adjust a resistance level of at least a portion of the resistance force profile through the input interface 146 of the wearable device 100 or a user terminal connected to the wearable device 100. That is, the user may adjust a resistance level of at least a portion of the resistance force profile to set an exercise method desired by the user. The resistance force profile will be described in detail with reference to FIG. 12.

Here, a neural network for each operation mode may be pretrained by a manufacturer of the wearable device 100. In addition, the neural network may be additionally trained, for example, fine-tuned, by the user of the wearable device 100. For example, the user may input feedback on a current output of the neural network to the wearable device 100, and the processor 142 may additionally train the neural network such that the feedback is reflected. For example, to train the neural network, backpropagation or reinforcement learning may be used, but examples are not limited thereto.

According to an example embodiment, the resistance level may be determined based on a position and an angle of a joint of the user. For example, a target pose of a left leg of the user may be set in advance, and a resistance level may be determined based on a difference between the target pose and a current pose of the user. In this example, when the difference between the target pose and the current pose is great, the resistance level may be determined to be small. In contrast, when the difference between the target pose and the current pose is small, the resistance level may be determined to be great. In addition, when the current pose corresponds to the target pose, a maximum resistance level may be provided to the user.

For example, in a case in which a pose of lifting a thigh by a preset or greater angle is set as the target pose, a resistance level may be greater when the user lifts the thigh higher, and the user may feel or experience a strongest resistance force from the target pose.

To determine a resistance level, a damping control technology to which a muscle model is applied may be used. When resistance levels determined through the damping control technology change in sequential order, a sense of resistance the user may feel or experience against a change in exercise load may be reduced.

According to a biological muscle model (e.g., a hill-type muscle model developed in medical engineering) that is generally known, when a stimulus signal is input to muscle, the input stimulus signal may be amplified in proportion to a force generated by the muscle (which is positive feedback), and muscular strength (or a force) amplified by the stimulus signal may have a relational expression by a length of the muscle and a contraction speed of the muscle.

When the muscle is contracted most and when the muscle is stretched most, there may not be a great force. However, when the muscle has an appropriate length, the muscle may have strongest power. A force generated based on a speed at which the length of the muscle changes may be greater when the speed of the change increases.

A relationship between the length of the muscle and the muscular strength of the muscle based on the length of the muscle may be in a form of a normal distribution. A relationship between the muscle stretching speed and the muscular strength of the muscle based on the muscle stretching speed may be in a form of a sigmoid.

The user may be familiar with a change in a physical movement based on the stretching of the muscle and the magnitude of the corresponding strength or force. Thus, when a resistance level corresponding to the magnitude of the force exhibited in response to the change in the physical movement is provided to the user, the user may also become familiar with a resistance force (or an exercise load) provided by the resistance level and a change in the resistance force based on a change in the resistance level.

The wearable device 100 may calculate a physical movement of the user based on a position and an angle of a joint of the user. In the exercise mode, the processor 142 of the wearable device 100 may calculate the physical movement and the magnitude of the corresponding force by the movement, based on the position and the angle of the joint of the user, and calculate a control level based on the calculated magnitude of the force.

In operation 1150, the processor 142 of the wearable device 100 determines a ratio between a time for which the motor driver circuit 112 is controlled to be a closed loop and a time for which the motor driver circuit 112 is controlled to be an open loop, based on the resistance level. Here, the ratio between the time for which the motor driver circuit 112 is controlled to be the closed loop and the time for which the motor driver circuit 112 is controlled to be the open loop may also be referred to as a connection ratio. For example, the connection ratio of the motor driver circuit 112 corresponding to the resistance level may be determined. The connection ratio of the motor driver circuit 112 may indicate a ratio at which the motor driver circuit 112 is controlled to be the closed loop (e.g., the first control state) or the open loop (e.g., the second control state) while energy is controlled not to be provided to the motor 114 from the battery 150. For example, based on a closed loop state, the connection ratio being 0.5 may indicate that the closed loop state is controlled to be 50% and an open loop state is controlled to be 50% in a set (or, alternatively, a preset) period. When the resistance level changes, the connection ratio may be dynamically adjusted.

For example, the connection ratio may be implemented using a PWM. However, examples are not limited to the foregoing example, and various methods may be applied to adjust a connection state of the motor driver circuit 112. When the PWM is used to control the connection ratio, the connection ratio determined in operation 1150 may be represented by a PWM having a certain duty ratio. The PWM having the duty ratio may be set (or, alternatively, preset) such that a desired resistance force is provided to the user. By the PWM having the duty ratio, the motor driver circuit 112 may be controlled to be the closed loop (first control state) or the open loop (second control state). For example, the processor 142 may control the motor driver circuit 112 to be the closed loop or the open loop by controlling an operation of each of switches in the motor driver circuit 112 using the PWM.

When the motor driver circuit 112 is the closed loop, the motor 114 may operate as a generator, and back-drivability of the motor 114 may decrease by dynamic braking. When the back-drivability decreases, a resistance force the user may feel or experience may increase. In contrast, when the motor driver circuit 112 is the open loop, the back-drivability of the motor 114 may increase, and only a frictional force by a gear ratio may be experienced by the user as a resistance force.

A desired back-drivability may be obtained by adjusting an open-closed state ratio of the motor driver circuit 112 based on the connection ratio. For example, the resistance force to be provided to the user may be adjusted by the ratio at which the motor driver circuit 112 is controlled to be the closed loop or the open loop within a uniform and repetitive time interval of the PWM, and the resistance force may increase when the ratio of the time for controlling the motor driver circuit 112 to be the closed loop increases.

In operation 1160, the processor 142 of the wearable device 100 controls the motor 114 through the motor driver circuit 112 based on the determined connection ratio. The motor driver circuit 112 may include at least one switch (e.g., the first to fourth switches 710-740) to be controlled based on the connection ratio, and the motor driver circuit 112 may be controlled to be the closed loop or the open loop by the switch. When the exercise mode is set as the operation mode of the wearable device 100, energy of the wearable device 100 may not be provided to the motor 114. For example, electric energy stored in the battery 150 of the wearable device 100 may not be provided to the motor 114. In this example, although the electric energy is not provided to the motor 114, the back-drivability of the motor 112 may be controlled by controlling the motor driver circuit 112 to be the closed loop or the open loop, and thus the resistance force may be adjusted by the controlled back-drivability.

When the user does not move, the motor 112 may not operate as the generator. When the motor 112 does not operate as the generator, the resistance force may not be generated and provided to the user. That is, the resistance force that is generated based on the back-drivability of the motor 112 may only occur when the user moves.

When the motor 112 operates as the generator, the battery 150 of the wearable device 100 may be charged based on energy generated by the generator. That is, while the wearable device 100 is operating in the exercise mode, the energy of the battery 150 of the wearable device 100 may be consumed considerably less, but charged instead. When the wearable device 100 operates in the exercise mode, the wearable device 100 may continue operating even when energy is not supplied from outside.

Operations 1140 through 1160 described above may be performed when the operation mode of the wearable device 100 is set as the exercise mode. Operations 1170 and 1180 to be described hereinafter may be performed when the operation mode of the wearable device 100 is set as the assistance mode.

In operation 1170, when the assistance mode is received as an input from the user, the processor 142 of the wearable device 100 calculates an assistance torque value for the joint based on the measured angle of the joint. For example, the assistance torque value for the input joint angle may be determined through the control algorithm. For another example, the assistance torque value may be output by inputting the joint angle to the neural network determined based on the operation mode.

According to an example embodiment, the processor 142 may determine a gait state or phase, or a progression of a gait cycle, of the user based on the angle of the joint, and determine an assistance torque value corresponding to the determined gait state or the determined progression of the gait cycle. For example, when the number of joints to be measured increases, a more accurate gait state or a more accurate progression of a gait cycle may be determined.

For example, the assistance torque value may be determined based on a desired (or, alternatively, a preset) torque profile. However, a method of calculating an assistance torque value is not limited to the foregoing example.

In operation 1180, the processor 142 of the wearable device 100 provides an assistance force to the user by controlling the motor 114 based on the assistance torque value. The wearable device 100 may operate or drive the motor 114 using the battery 150 of the wearable device 100 such that an assistance torque is output, and provide the assistance force to the user by the assistance torque output by the motor 114. The assistance torque value may indicate a control signal applied to the motor 114, and the assistance torque may indicate a rotation torque output by the motor 114 based on the assistance torque value. The assistance force may indicate a force the user may feel or experience by the assistance torque.

Although the wearable device 100 is described with reference to FIG. 11 as providing both the exercise mode and the assistance mode to a user, the wearable device 100 may operate only in the exercise mode. In a case in which the wearable device 100 operates only in the exercise mode, operations 1110, 1130, 1170, and 1180 described above may not be performed. In addition, the wearable device 100 may not include a battery to provide power to the motor 114. When the battery is not included, the wearable device 100 may be lightened in terms of weight.

FIG. 12 is a diagram illustrating an example of a resistance force profile output from a user terminal according to at least one example embodiment.

Referring to FIG. 12, the wearable device 100 may be connected to a user terminal 1200 through a wired or wireless network. For example, the wearable device 100 may transmit and receive information associated with the wearable device 100 through an application installed in the user terminal 1200. The information associated with the wearable device 100 may include, for example, a setting value of the wearable device 100, an operation state of the wearable device 100, a device state of the wearable device 100, and the like. The setting values of the wearable device 100 may include, for example, detailed setting values that are set by a user for an assistance mode or an exercise mode. The operation state of the wearable device 100 may include, for example, a current gait state of the user or a progression of a gait cycle. The device state of the wearable device 100 may include, for example, a residual amount of the battery 150.

Various resistance force profiles associated with the exercise mode may be stored in advance in the wearable device 100 or the user terminal 1200. For example, the resistance force profiles may be generated in advance to produce different exercise effects.

The user may individualize an existing resistance force profile 1210 by adjusting at least a portion 1220 of the resistance force profile 1210 to be a resistance level desired by the user. For example, the portion 1220 may correspond to a swing state, and the user may adjust the resistance level of the portion 1220 such that the resistance level is to be almost minimized in the swing state. For example, the user may adjust the resistance level by touching the portion 1220 through a touch panel of the user terminal 1200 and dragging a trajectory of the selected portion 1220.

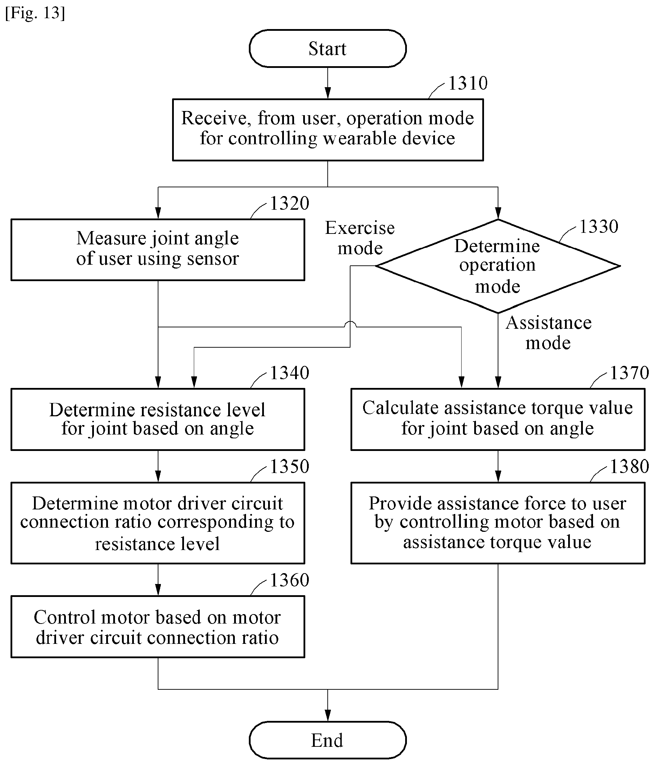

FIG. 13 is a flowchart illustrating another example of a method of providing a resistance force according to at least one example embodiment.

Operations 1310 through 1380 to be described hereinafter with reference to FIG. 13 may be performed by the wearable device 100.

Referring to FIG. 13, in operation 1310, the wearable device 100 receives, from a user, an operation mode that controls the wearable device 100. For a more detailed description of operation 1310, reference may be made to the description of operation 1110 provided above with reference to FIG. 11.

In operation 1320, the wearable device 100 measures an angle of a joint of the user using the sensor 121. For a more detailed description of operation 1320, reference may be made to the description of operation 1120 provided above with reference to FIG. 11. Operation 1320 may be independently performed with operation 1330 in parallel.

In operation 1330, the wearable device 100 determines whether the operation mode is an exercise mode or an assistance mode.

For example, in a case in which the wearable device 100 operates based on a plurality of neural networks, the wearable device 100 may determine a neural network corresponding to the determined operation mode. Based on the determined neural network, subsequent operations may be performed. For example, an exercise mode neural network may be determined for the exercise mode, and an assistance mode neural network may be determined for the assistance mode. When the operation mode is the exercise mode, operations 1340 through 1360 may be performed. When the operation mode is the assistance mode, operations 1370 and 1380 may be performed.

Although it is described above that the exercise mode or the assistance mode operates based on its corresponding neural network, examples are not limited thereto. For example, an operation of the wearable device 100 may be controlled through a control algorithm, not such a neural network.

### <Exercise Mode>

In operation 1340, the processor 142 of the wearable device 100 determines a resistance level for the joint based on the measured angle of the joint. For a more detailed description of operation 1340, reference may be made to the description of operation 1140 provided above with reference to FIG. 11.

In operation 1350, the processor 142 of the wearable device 100 determines a connection ratio of the motor driver circuit 112 corresponding to the resistance level. For a more detailed description of operation 1350, reference may be made to the description of operation 1150 provided above with reference to FIG. 11.

In operation 1360, the processor 142 of the wearable device 100 controls the motor 114 through the connection ratio of the motor driver circuit 112. For a more detailed description of operation 1360, reference may be made to the description of operation 1160 provided above with reference to FIG. 11.

### <Assistance Mode>

In operation 1370, the processor 142 of the wearable device 100 calculates an assistance torque value for the joint based on the measured angle of the joint. For a more detailed description of operation 1370, reference may be made to the description of operation 1170 provided above with reference to FIG. 11.

In operation 1380, the processor 142 of the wearable device 100 provides an assistance force to the user by controlling the motor 114 based on the assistance torque value. For a more detailed description of operation 1380, reference may be made to the description of operation 1180 provided above with reference to FIG. 11.

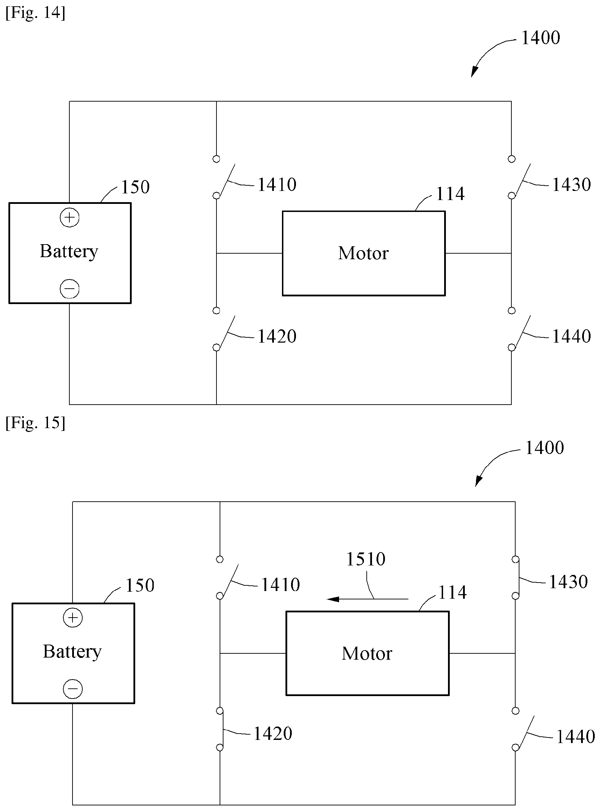

FIG. 14 is a diagram illustrating an example of a motor driver circuit of an open loop according to at least one example embodiment.

Referring to FIG. 14, a motor driver circuit 1400 may be an H-bridge circuit. The motor driver circuit 1400 may have an open or closed state that is determined based on a connection state of switches 1410 through 1440. The switches 1410 through 1440 may be embodied as, for example, a bipolar junction transistor (BJT) and a metal-oxide-semiconductor field-effect transistor (MOSFET), but examples are not limited thereto.

When the motor driver circuit 1400 is in an open circuit state, dynamic braking of the motor 114 may be minimized, and thus back-drivability of the motor 114 may increase. In such a case, the back-drivability may be a frictional force generated by gears connected to the motor 114.

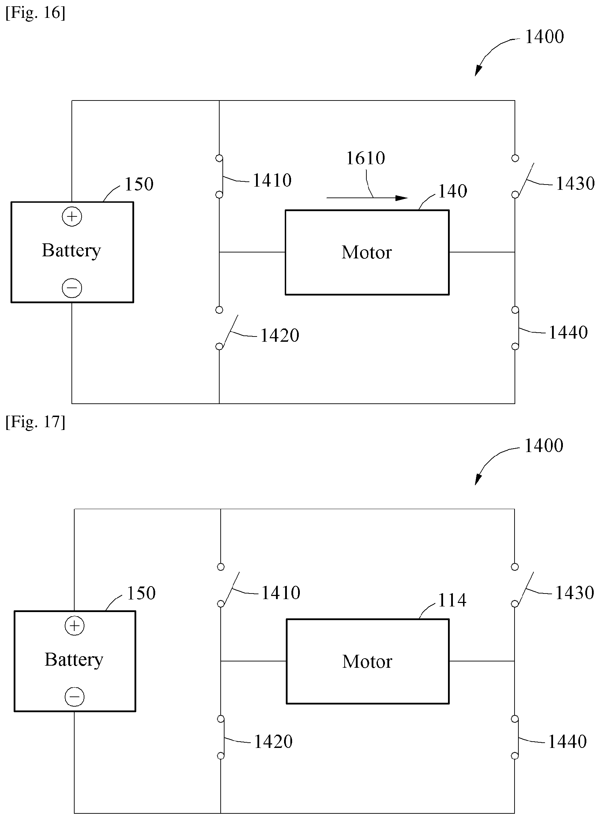

FIGS. 15 and 16 are diagrams illustrating examples of a motor driver circuit of a closed loop according to at least one example embodiment.

According to an example embodiment, a closed loop of the motor driver circuit 1400 may be formed to include the battery 150 and the motor 114.

For example, when the switches 1410 and 1440 of the motor driver circuit 1400 are open and the switches 1420 and 1430 of the motor driver circuit 1400 are closed, a current provided to the motor 114 may flow in a first direction 1510.

For another example, when the switches 1410 and 1440 are closed and the switches 1420 and 1430 are open, a current provided to the motor 114 may flow in a second direction 1610. The second direction 1610 may be a direction opposite to the first direction 1510. Based on such a current direction, a rotation direction of an axis of the motor 114 may change.

These example closed loops may be formed in a case of an assistance mode, and the example current directions 1510 and 1610 may be determined based on a direction of an assistance force to be provided to a user.

FIG. 17 is a diagram illustrating an example of a motor driver circuit of a closed loop according to at least one example embodiment.

Referring to FIG. 17, the motor driver circuit 1400 connected to the motor 114 that is controlled not to use energy of the battery 150 may form a closed loop. That is, in the example of FIG. 17, the motor driver circuit 1400 may be a motor driver circuit for an exercise mode that does not use power of the battery 150 and a closed loop circuit connected to the motor 114.

For example, when the switches 1410 and 1430 of the motor driver circuit 1400 are open and the switches 1420 and 1440 of the motor driver circuit 1400 are closed, a closed loop including the motor 114 may be formed. That is, the closed loop may be formed by a lower driver circuit of the motor driver circuit 1400. The closed loop may be formed in a case of the exercise mode, and dynamic braking of the motor 114 may occur. A user who rotates the motor 114 may feel or experience a resistance force by the dynamic braking.

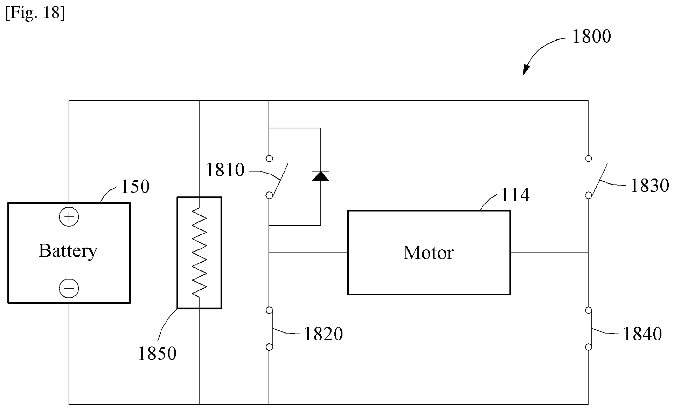

FIG. 18 is a diagram illustrating an example of a motor driver circuit of a closed loop including a braking resistor according to at least one example embodiment.

Referring to FIG. 18, a motor driver circuit 1800 includes the battery 150, switches 1810 through 1840 connected to the motor 114, and a braking resistor 1850. The switches 1820 and 1840 are closed, and thus a closed loop including the motor 114 may be formed. When the closed loop is formed, a resistance force that may be felt or experienced by a user may be maximized.

When a ratio of an open loop state and a closed loop state of the motor driver circuit 1800 is adjusted by a connection ratio, a level of the resistance force may be adjusted. The closed loop state may be maintained to maximize the resistance force, and the open loop state may be maintained to minimize the resistance force.

For example, in a state in which the motor driver circuit 1800 is controlled to be the closed loop, the motor 114 may operate as a generator for an external force by the user, and generated energy may be consumed as heat through the braking resistor 1850. For another example, in the motor driver circuit 1800, a current which is the energy generated by the motor 114 may flow in a direction from a (+) terminal of the battery 150 to a (-) terminal of the battery 150 by a diode, regardless of a rotation direction of a rotation axis of the motor 114. Thus, the generated energy may charge the battery 150.

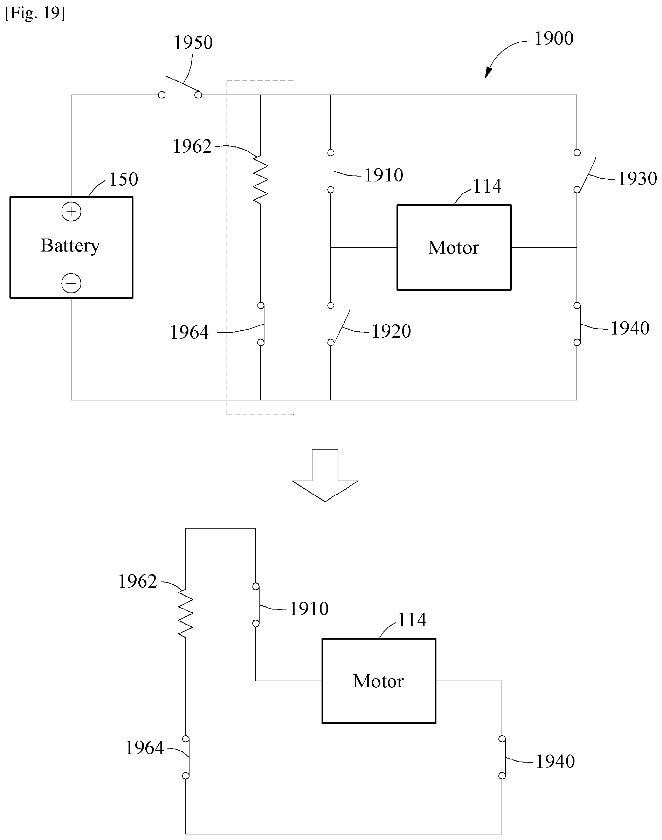

FIG. 19 is a diagram illustrating an example of a motor driver circuit of a closed loop including a resistor according to at least one example embodiment.

Referring to FIG. 19, a motor driver circuit 1900 obtained by adding an auxiliary path including at least one resistor 1962 and switch 1964 to the motor driver circuit 1400 described above with reference to FIGS. 14 through 17 may increase electrical stability of a closed loop.

In the motor driver circuit 1900 in a closed loop state including the motor 114, the motor 114 may operate as a generator while rotating by an external force and generate an electromotive force. By the generated electromotive force, there may be a probability that electronic components in the closed loop are damaged. Thus, by adding the resistor 1962 to the closed loop, an internal resistance value of the closed loop may increase, and thus a magnitude of a current generated by the electromotive force may decrease. As the magnitude of the current decreases, the probability that the internal electronic components in the closed loop are damaged may also be reduced.

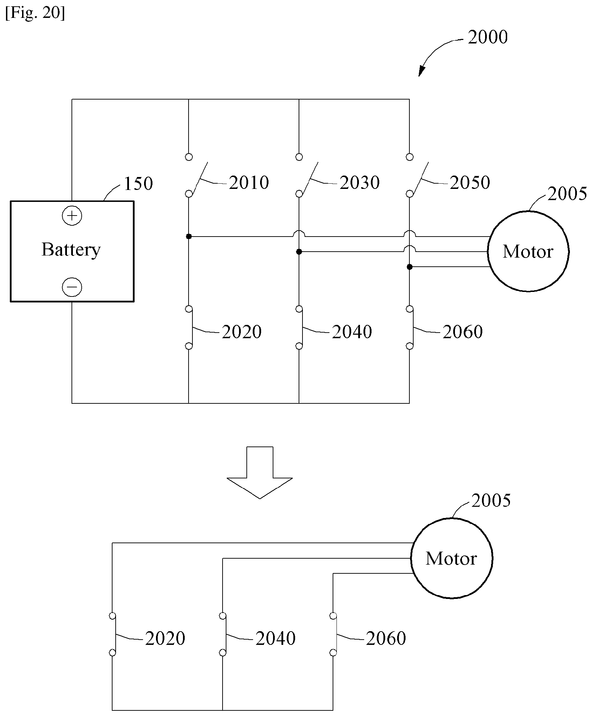

FIG. 20 s a diagram illustrating an example of a motor driver circuit of a closed loop including a brushless direct current (BLDC) motor according to at least one example embodiment.

The motor driver circuits 1400, 1800, and 1900 illustrated in FIGS. 14 through 19 may be connected to a direct circuit (DC) motor. Referring to FIG. 20, as another example, a motor driver circuit 2000 may be connected to a BLDC motor 2005. The BLDC motor 2005 may be connected to three terminals in the motor driver circuit 2000. In general, compared to a DC motor, a BLDC motor may generate a torque that is great relative to a volume, and may not have a friction generated between a brush and a rotor coil because it does not use a mechanical brush that is used in the DC motor for commuting a current. Since there is no friction between the brush and the rotor coil, the BLDC motor may have a greater durability than the DC motor.

For example, when switches 2010, 2030, and 2050 of the motor driver circuit 2000 are open, and switches 2020, 2040, and 2060 of the motor driver circuit 2000 are closed, a closed loop including the BLDC motor 2005 may be formed. The closed loop may be formed with the battery 150 excluded.

As a PWM signal is applied to the switches 2020, 2040, and 2060, a closed loop state and an open loop state of the motor driver circuit 2000 may be controlled. Here, a control ratio may change according to a resistance level.

The switch 2020 may control opening and closing of a first terminal (u) of the BLDC motor 2005. The switch 2040 may control opening and closing of a second terminal (v) of the BLDC motor 2005. The switch 2060 may control opening and closing of a third terminal (w) of the BLDC motor 2005. For example, when the same PWM signal is applied to the switches 2020, 2040, and 2060, a closed loop that is not related to a commutation sequence for the BLDC motor 2005 may be formed.

Since the BLDC motor 2005 are connected to the three terminals u, v, and w, it may possible to form a closed loop by determining hall sensor information (e.g., an angle of a rotation axis of a motor) of a rotor and then selectively connecting two switches among the switches 2020, 2040, and 2060 to match the commutation sequence, in order to form an electrical closed loop among the terminals u, v, and w. However, in a case in which the BLDC motor 2005 is controlled to control only back-drivability of the BLDC motor 2005 in an exercise mode, it is possible to form a closed loop by connecting all the switches 2020, 2040, and 2060, without considering the commutation sequence. In such a case, there is no need to consider the commutation sequence to form the closed loop, a time needed for calculating the commutation sequence may be reduced and a probability of a malfunction of the BLDC motor 2005 may also be reduced.

For another example, a PWM signal may be applied to each of the switches 2020, 2040, and 2060 to form a closed loop corresponding to a commutation sequence for a state classified based on an angle of a rotation axis of the BLDC motor 2005.

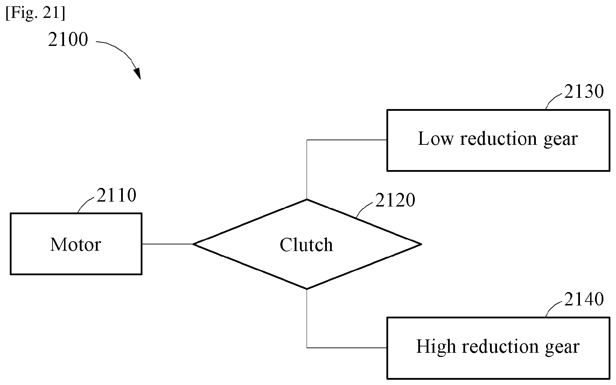

FIG. 21 is a diagram illustrating an example of a driver of a wearable device according to at least one example embodiment.

Referring to FIG. 21, the driver 110 of the wearable device 100 includes a motor 2110, a clutch 2120, and a plurality of gears including a low reduction gear 2130 and a high reduction gear 2140. From among the gears, different gears may be selected according to an input of a user or a determined resistance level. The clutch 2120 may control driving force transfer by selectively connecting the motor 2110 to one of the gears.

A gear ratio may be differently set based on an objective of an operation mode of the wearable device 100, and thus a magnitude of a resistance force to be provided to the user may be adjusted.

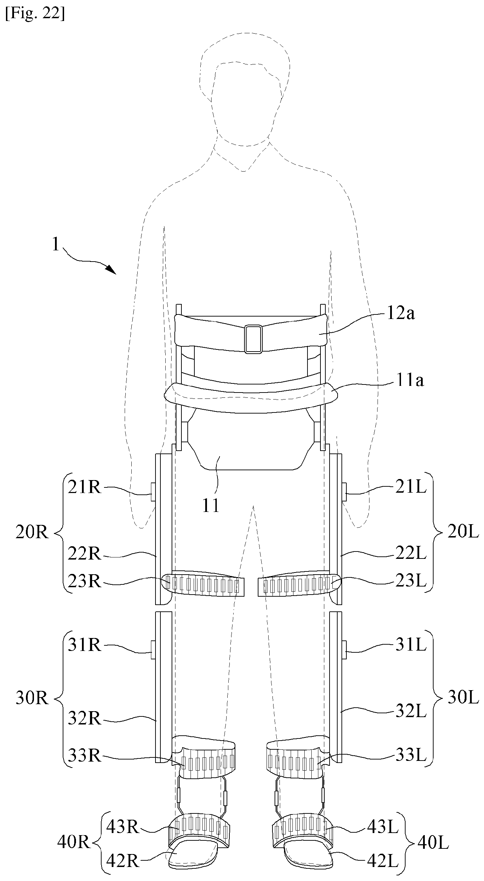

Unlike the hip-type wearable device 100 described above with reference to FIGS. 1A through 1D, a wearable device may be a whole body-type wearable device 1 to be described hereinafter with reference to FIGS. 22 through 24. The whole body-type wearable device 1 may be configured to provide a walking assistance torque respectively to a hip joint, a knee joint, and an ankle joint of a user.

### <Overview of whole body-type walking assist device>

FIGS. 22 through 24 are diagrams illustrating an example of a whole body-type wearable device 1 according to at least one example embodiment.

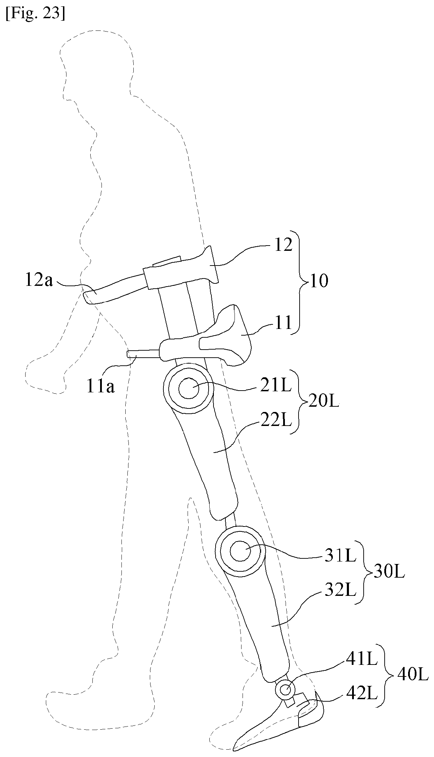

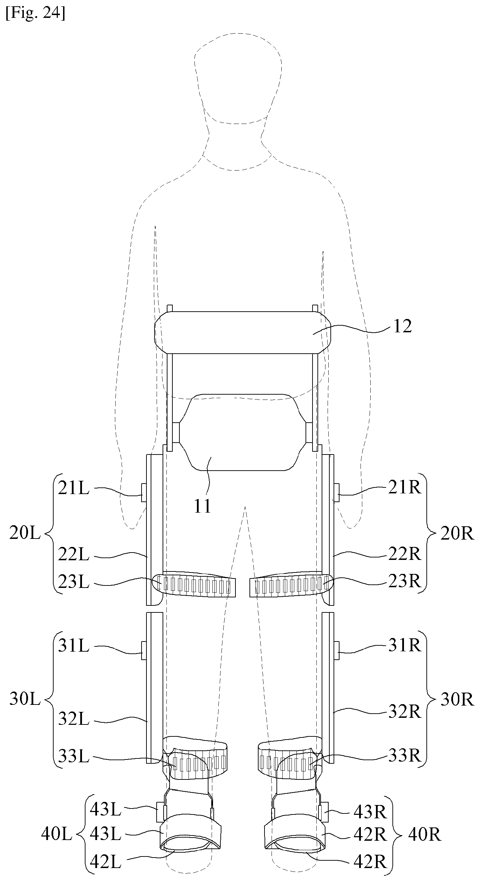

FIG. 22 is a front view of the whole body-type wearable device 1, FIG. 23 is a side view of the whole body-type wearable device 1, and FIG. 24 is a rear view of the whole body-type wearable device 1.

According to an example embodiment, the whole body-type wearable device 1 may include the driver 110, the sensor 120, the IMU 130, the controller 140, and the battery 150 that are described above.

Referring to FIGS. 22 through 24, the whole body-type wearable device 1 may be provided in an exoskeleton structure to be worn on a left leg and a right leg of a user. With the wearable device 1 on, the user may be able to perform various movements, for example, extension, flexion, adduction, and abduction. The extension may indicate a movement or an exercise of stretching joints, and the flexion may indicate a movement or an exercise of bending joints. The adduction may indicate a movement or an exercise of moving legs to be closer to a central axis of a body, and the abduction may indicate a movement or an exercise of stretching legs in a direction receding from a central axis of a body.

Referring to FIGS. 22 through 24, the wearable device 1 includes a main body portion 10 and a device portion including 20R and 20L, 30R and 30L, and 40R and 40L.

The main body portion 10 includes a housing 11 in which various components are embedded. The components to be embedded in the housing 11 may include, for example, a central processing unit (CPU), a printed circuit board (PCB), various types of storage devices, and a power source. Although not illustrated, the whole body-type wearable device 1 may also include the driver 110, the sensor 120, the IMU 130, and the controller 140 that are described above. For example, the main body portion 10 may include the controller 140, where the controller 140 may include the CPU and the PCB.

The CPU may be a microprocessor. The microprocessor may be provided with an arithmetic logic operator, a register, a program counter, an instruction decoder, and/or a control circuit in a silicon chip. The CPU may select a control mode that is suitable for a walking environment, and generate a control signal to control an operation of the device portion.

The PCB refers to a board on which a circuit is printed, and the CPU and/or the various storage devices may be installed in the PCB. The PCB may be fixed to an inner side surface of the housing 11.

The various types of storage devices may be embedded in the housing 11. The storage devices may include a magnetic disk storage device configured to magnetize a magnetic disk surface and store data, and a semiconductor memory device configured to store data using various types of memory semiconductors.

The power source embedded in the housing 11 may supply power to the various components embedded in the housing 11 or the device portion.

The main body portion 10 further includes a waist support 12 configured to support a waist of the user. The waist support 12 may be provided in a shape of a curved flat surface plate that supports the waist of the user.

The main body portion 10 further includes a fixing portion 11a configured to fix the housing 11 to a hip portion of the user, and a fixing portion 12a configured to fix the waist support 12 to the waist of the user. The fixing portions 11a and 12a may be embodied by one of a band, a belt, and a strap which are elastic.

The main body portion 10 also includes the IMU 130. For example, the IMU 130 may be provided inside or outside the housing 11. The IMU 130 may be provided on the PCB provided inside the housing 11. The IMU 130 may measure an acceleration and an angular velocity.

The device portion includes a first structure 20, a second structure 30, and a third structure 40 as illustrated in FIGS. 22 through 24.

The first structure 20 including right 20R and left 20L portions may assist or support a movement of a thigh and a hip joint of the user when the user walks. The first structure 20 includes a first driving device including 21R and 21L, a first support including 22R and 22L, and a first fixing portion including 23R and 23L.

The driver 110 may include the first driving device including 21R and 21L. The description of the driver 110 may be replaced with the description of the first driving device including 21R and 21L to be provided hereinafter with reference to FIGS. 22 through 24.

The first driving device including 21R and 21L may be disposed on the hip joint of the first structure 20 including 20R and 20L, and generate a rotational force of different magnitudes in a certain direction. The rotational force generated in the first driving device including 21R and 21L may be applied to the first support including 22R and 22L. The first driving device including 21R and 21L may be set to rotate within a motion range of a hip joint of a human body.

The first driving device including 21R and 21L may operate based on a control signal provided by the main body portion 10. The first driving device including 21R and 21L may be embodied by one of a motor, a vacuum pump, and a hydraulic pump, but examples are not limited thereto.

A joint angle sensor may be provided around the first driving device including 21R and 21L. The joint angle sensor may detect an angle by which the first driving device including 21R and 21L rotates on a rotation axis. The sensor 120 may include the joint angle sensor.

The first support including 22R and 22L may be physically connected to the first driving device including 21R and 21L. The first support including 22R and 22L may rotate in a certain direction by the rotational force generated from the first driving device including 21R and 21L.

The first support including 22R and 22L may be provided in various shapes. For example, the first support including 22R and 22L may be provided in a shape in which nodes are connected with each other. A joint may be between the nodes, by which the first support including 22R and 22L may be bent within a certain range. For another example, the first support including 22R and 22L may be provided in a shape of a rod. In this example, the first support including 22R and 22L may be formed with a flexible material that may be bent within a certain range.

The first fixing portion including 23R and 23L may be provided in the first support including 22R and 22L. The first fixing portion including 23R and 23L may fix the first support including 22R and 22L to the thigh of the user.

FIGS. 22 through 24 illustrate that the first support including 22R and 22L is fixed on an outer side of the thigh of the user by the first fixing portion including 23R and 23L. When the first driving device including 21R and 21 L operates and the first support including 22R and 22L rotates, the thigh to which the first support including 22R and 22L is fixed may also rotate in a same direction as a rotation direction in which the first support including 22R and 22L rotates.

The first fixing portion including 23R and 23L may be embodied by one of a band, a belt, and a strap which are elastic, or by a metal material. FIG. 22 illustrates the first fixing portion including 23R and 23L as a chain.

The second structure 30 including right 30R and left 30L portions may assist or support a movement of a calf and a knee joint of the user when the user walks. The second structure 30 including 30R and 30L includes a second driving device including 31R and 31L, a second support including 32R and 32L, and a second fixing portion including 33R and 33L.

The second driving device including 31R and 31L may be disposed on the knee joint of the second structure 30 including 30R and 30L, and generate a rotational force of different magnitudes in a certain direction. The rotational force generated in the second driving device including 31R and 31L may be applied to the second support including 32R and 32L. The second driving device including 31R and 31L may be set to rotate within a motion range of a knee joint of a human body.

The driver 110 may include the second driving device including 31R and 31L. Here, what has been described above in relation to a hip joint with reference to FIGS. 1A through 1D may be similarly and substantially applied to the knee joint.

The second driving device including 31R and 31L may operate based on a control signal provided by the main body portion 10. The second driving device including 31R and 31L may be embodied by one of a motor, a vacuum pump, and a hydraulic pump, but examples are not limited thereto.

A joint angle sensor may be provided around the second driving device including 31R and 31L. The joint angle sensor may detect an angle by which the second driving device including 31R and 31L rotates on a rotation axis. The sensor 120 may include the joint angle sensor.

The second support including 32R and 32L may be physically connected to the second driving device including 31R and 31L. The second support including 32R and 32L may rotate in a direction by the rotational force generated from the second driving device 31R and 31L.

The second fixing portion including 33R and 33L may be provided in the second support including 32R and 32L. The second fixing portion including 33R and 33L may fix the second support including 32R and 32L to the calf of the user. FIGS. 22 through 24 illustrate that the second support including 32R and 32L is fixed on an outer side of the calf of the user by the second fixing portion including 33R and 33L. When the second driving device including 31R and 31L operates and the second support including 32R and 32L rotates, the calf to which the second support including 32R and 32L is fixed may also rotate in a same direction as a rotation direction in which the second support including 32R and 32L rotates.

The second fixing portion including 33R and 33L may be embodied by one of a band, a belt, and a strap which are elastic, or by a metal material.

The third structure 40 including right 40R and left 40L portions may assist or support a movement of an ankle joint and related muscles of the user when the user walks. The third structure 40 including 40R and 40L includes a third driving device including 41R and 41L, a foot support including 42R and 42L, and a third fixing portion including 43R and 43L.

The driver 110 may include the third driving device including 41R and 41L. Here, what has been described above in relation to a hip joint withe reference to FIGS. 1A through 1D may be similarly and substantially applied to the ankle joint.

The third driving device including 41R and 41L may be disposed on the ankle joint of the third structure 40 including 40R and 40L, and operate based on a control signal provided by the main body portion 10. Similar to the first driving device including 21R and 21L or the second driving device including 30R and 30L, the third driving device including 41R and 41L may be embodied by a motor.

The foot support including 42R and 42L may be disposed at a position corresponding to a sole of a foot of the user, and physically connected to the third driving device including 41R and 41L.

The third fixing portion including 43R and 43R may be provided in the foot support including 42R and 42L. The third fixing portion including 43R and 43L may fix the foot of the user to the foot support including 42R and 42L.

The units and/or modules described herein may be implemented using hardware components and software components. For example, the hardware components may include microphones, amplifiers, band-pass filters, audio to digital convertors, and processing devices. A processing device may be implemented using one or more hardware device configured to carry out and/or execute program code by performing arithmetical, logical, and input/output operations. The processing device(s) may include a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The software may include a computer program, a piece of code, an instruction, or some combination thereof, to independently or collectively instruct and/or configure the processing device to operate as desired, thereby transforming the processing device into a special purpose processor. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher level code that may be executed by the computer using an interpreter. The above-described devices may be configured to act as one or more software modules in order to perform the operations of the above-described example embodiments, or vice versa.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components. The invention is defined by the following claims.

## Claims

1. A method of controlling a motor driver circuit (112; 1400; 1800; 1900) of wearable device (100), the method comprising:
controlling a first switch (710; 1410; 1810; 1950) and a second switch (720; 1430; 1830; 1930) of a motor driver circuit (112; 1400; 1800; 1900) so that a battery (150) does not form a closed loop through the motor driver circuit (112; 1400; 1800; 1900) in response to an exercise mode being set in the wearable device (100);
determining (1140; 1340) a resistance level to apply to a first joint of a user;
determining (1150; 1350) a connection ratio between a connected time for which terminals of a motor (114) are to be electrically connected in a closed loop and a disconnected time for which the terminals of the motor are to be electrically disconnected, based on the resistance level; and
controlling (1160; 1360) a third switch (730; 1420; 1820; 1910) and a fourth switch (740;1440; 1840; 1940) of the motor driver circuit (112; 1400; 1800; 1900) electrically connected to the motor (114) based on the connection ratio.

2. The method of claim 1, wherein the connection ratio is represented by a pulse width modulation, PWM.

3. The method of claim 2, wherein a resistance level to be provided to the user is adjusted based on the connection ratio such that, as the connected time for which the terminals of the motor (114) are to be electrically connected in the closed loop increases, the resistance force increases.

4. The method of claim 1, wherein, in a state in which the terminals of the motor (114) are to be electrically connected in the closed loop, the motor (114) is configured to operate as a generator with respect to an external force by the user.

5. The method of claim 4, further comprising:
charging the battery (150) of the wearable device based on energy generated by the generator, when the motor (114) operates as the generator.

6. The method of claim 1, further comprising:
receiving, from the user, an instruction to set an operation mode of the wearable device to the exercise mode.

7. The method of claim 6, wherein, when the exercise mode is set, the motor (114) is not provided with energy from the battery (150) of the wearable device.

8. The method of claim 1, further comprising:
receiving, from the user, an instruction to set an operation mode of the wearable device to an assistance mode;
calculating an assistance torque value to apply to the first joint in the assistance mode based on a first angle; and
providing an assistance force to the user by controlling the motor based on the assistance torque value.

9. A non-transitory computer-readable medium comprising computer readable instructions to cause a computer to perform the method of claim 1 on a wearable device comprising a motor (114), a motor driver circuit (112; 1400; 1800; 1900), a first switch (710; 1410; 1810; 1950), a second switch (720; 1430; 1830; 1930), a third switch (730; 1420; 1820; 1910), a fourth switch (740; 1440; 1840; 1940) and a processor configured to perform the method of claim 1.

10. A wearable device (100) configured to control a motor driver circuit (112; 1400; 1800; 1900) of the wearable device (100), the wearable device (100) comprising:
a memory (144) configured to store a program that includes instructions to provide the resistance force to the user ;
a motor driver circuit (112; 1400; 1800; 1900);
a motor (114) electrically connected to the motor driver circuit (112; 1400; 1800; 1900); and
a processor (142) configured to execute the program to,
control a first switch (710; 1410; 1810; 1950) and a second switch (720; 1430; 1830; 1930) of the motor driver circuit (112; 1400; 1800; 1900) so that a battery (150) does not form a closed loop through the motor driver circuit (112; 1400; 1800; 1900) in response to an exercise mode being set in the wearable device (100),
determine (1140; 1340) a resistance level to apply to a first joint of the user,
determine (1150; 1350 ) a connection ratio between a connected time for which terminals of a motor (114) are to be electrically connected in a closed loop and a disconnected time for which the terminals of the motor are to be electrically disconnected, based on the resistance level, and
control (1160; 1360) a third switch (730; 1420; 1820; 1910) and a fourth switch (740;1440; 1840; 1940) of the motor driver circuit (112; 1400; 1800; 1900) electrically connected to the motor (114) based on the connection ratio.

11. The wearable device of claim 10, wherein the connection ratio is represented by a pulse width modulation, PWM, and
a resistance force to be provided to the user is adjusted based on the connection ratio such that, as the connected time for which the terminals of the motor (114) are to be electrically connected in the closed loop increases, the resistance force increases.

12. The wearable device of claim 10, wherein, in a state in which the terminals of the motor (114) are to be the electrically connected in closed loop, the motor (114) is configured to operate as a generator with respect to an external force by the user.

13. The wearable device of claim 10, wherein the processor (142) is further configured to,
receive, from the user, an instruction to set an operation mode of the wearable device to the exercise mode, and
determine the resistance level to apply to the first joint.

14. The wearable device of claim 10, wherein the processor (142) is further configured to,
receive, from the user, an instruction to set an operation mode of the wearable device to an assistance mode;
calculate an assistance torque value to apply to the first joint in the assistance mode based on a first angle; and
provide an assistance force to the user by controlling the motor based on the assistance torque value.

## Patentansprüche

1. Verfahren zum Steuern einer Motortreiberschaltung (112; 1400; 1800; 1900) eines tragbaren Geräts (100), wobei das Verfahren Folgendes umfasst:
Steuern eines ersten Schalters (710; 1410; 1810; 1950) und eines zweiten Schalters (720; 1430; 1830; 1930) einer Motortreiberschaltung (112; 1400; 1800; 1900), derart, dass eine Batterie (150) in Reaktion auf einen in dem tragbaren Gerät (100) eingestellten Trainingsmodus keine geschlossene Schleife über die Motortreiberschaltung (112; 1400; 1800; 1900) bildet;
Bestimmen (1140; 1340) eines Widerstandspegels, der auf ein erstes Gelenk eines Benutzers anzuwenden ist;
Bestimmen (1150; 1350) eines Verbindungsverhältnisses zwischen einer Verbindungszeit, während der Anschlüsse eines Motors (114) in einer geschlossenen Schleife elektrisch verbunden sein sollen, und einer Trennungszeit, während der die Anschlüsse des Motors elektrisch getrennt sein sollen, basierend auf dem Widerstandspegel; und
Steuern (1160; 1360) eines dritten Schalters (730; 1420; 1820; 1910) und eines vierten Schalters (740; 1440; 1840; 1940) der Motortreiberschaltung (112; 1400; 1800; 1900), die elektrisch mit dem Motor (114) verbunden ist, auf der Grundlage des Verbindungsverhältnisses.

2. Verfahren nach Anspruch 1, wobei das Verbindungsverhältnis durch eine Pulsweitenmodulation (PWM) dargestellt wird.

3. Verfahren nach Anspruch 2, wobei ein dem Benutzer bereitzustellender Widerstandspegel auf der Grundlage des Verbindungsverhältnisses so eingestellt wird, dass die Widerstandskraft zunimmt, wenn die Verbindungszeit, während der die Anschlüsse des Motors (114) in der geschlossenen Schleife elektrisch verbunden sein sollen, zunimmt.

4. Verfahren nach Anspruch 1, wobei in einem Zustand, in dem die Anschlüsse des Motors (114) in einer geschlossenen Schleife elektrisch verbunden sein sollen, der Motor (114) so konfiguriert ist, dass er in Bezug auf eine vom Benutzer ausgeübte äußere Kraft als Generator arbeitet.

5. Verfahren nach Anspruch 4, umfassend ferner:
Laden der Batterie (150) des tragbaren Geräts auf der Grundlage der vom Generator erzeugten Energie, wenn der Motor (114) als Generator arbeitet.

6. Verfahren nach Anspruch 1, umfassend ferner:
Empfangen einer Anweisung vom Benutzer, einen Betriebsmodus des tragbaren Geräts auf den Trainingsmodus einzustellen.

7. Verfahren nach Anspruch 6, wobei, wenn der Trainingsmodus eingestellt ist, der Motor (114) nicht mit Energie aus der Batterie (150) des tragbaren Geräts versorgt wird.

8. Verfahren nach Anspruch 1, umfassend ferner:
Empfangen einer Anweisung vom Benutzer, einen Betriebsmodus des tragbaren Geräts auf einen Unterstützungsmodus einzustellen;
Berechnen eines Unterstützungsdrehmomentwerts, der im Unterstützungsmodus auf das erste Gelenk anzuwenden ist, basierend auf einem ersten Winkel; und
Bereitstellen einer Unterstützungskraft für den Benutzer durch Steuern des Motors basierend auf dem Unterstützungsdrehmomentwert.

9. Nichtflüchtiges, computerlesbares Medium, das computerlesbare Anweisungen umfasst, um einen Computer dazu zu veranlassen, das Verfahren nach Anspruch 1 auf einem tragbaren Gerät auszuführen, das einen Motor (114), eine Motortreiberschaltung (112; 1400; 1800; 1900), einen ersten Schalter (710; 1410; 1810; 1950), einen zweiten Schalter (720; 1430; 1830; 1930), einen dritten Schalter (730; 1420; 1820; 1910), einen vierten Schalter (740; 1440; 1840; 1940) und einen Prozessor umfasst, der zum Ausführen des Verfahrens nach Anspruch 1 konfiguriert ist.

10. Tragbares Gerät (100), das zum Steuern einer Motortreiberschaltung (112; 1400; 1800; 1900) des tragbaren Geräts (100) konfiguriert ist, wobei das tragbare Gerät (100) Folgendes umfasst:
einen Speicher (144), der zum Speichern eines Programms konfiguriert ist, das Anweisungen zum Bereitstellen der Widerstandskraft für den Benutzer enthält;
eine Motortreiberschaltung (112; 1400; 1800; 1900);
einen Motor (114), der elektrisch mit der Motortreiberschaltung (112; 1400; 1800; 1900) verbunden ist; und
einen Prozessor (142), der dazu konfiguriert ist, das Programm auszuführen, um
einen ersten Schalter (710; 1410; 1810; 1950) und einen zweiten Schalter (720; 1430; 1830; 1930) der Motortreiberschaltung (112; 1400; 1800; 1900) so zu steuern, dass eine Batterie (150) in Reaktion auf einen in dem tragbaren Gerät (100) eingestellten Trainingsmodus keine geschlossene Schleife über die Motortreiberschaltung (112; 1400; 1800; 1900) bildet,
einen Widerstandspegel, der auf ein erstes Gelenk des Benutzers anzuwenden ist, zu bestimmen (1140; 1340),
ein Verbindungsverhältnis zwischen einer Verbindungszeit, während der Anschlüsse eines Motors (114) in einer geschlossenen Schleife elektrisch verbunden sein sollen, und einer Trennungszeit, während der die Anschlüsse des Motors elektrisch getrennt sein sollen, auf der Grundlage des Widerstandspegels zu bestimmen (1150; 1350), und
einen dritten Schalter (730; 1420; 1820; 1910) und einen vierten Schalter (740; 1440; 1840; 1940) der Motortreiberschaltung (112; 1400; 1800; 1900), die elektrisch mit dem Motor (114) verbunden ist, auf der Grundlage des Verbindungsverhältnisses zu steuern (1160; 1360).

11. Tragbares Gerät nach Anspruch 10, wobei das Verbindungsverhältnis durch eine Pulsweitenmodulation (PWM) dargestellt wird und
eine dem Benutzer bereitzustellende Widerstandskraft auf der Grundlage des Verbindungsverhältnisses so eingestellt wird, dass die Widerstandskraft zunimmt, wenn die Verbindungszeit, während der die Anschlüsse des Motors (114) in der geschlossenen Schleife elektrisch verbunden sein sollen, zunimmt.

12. Tragbares Gerät nach Anspruch 10, wobei in einem Zustand, in dem die Anschlüsse des Motors (114) in einer geschlossenen Schleife elektrisch verbunden sein sollen, der Motor (114) so konfiguriert ist, dass er in Bezug auf eine vom Benutzer ausgeübte äußere Kraft als Generator arbeitet.

13. Tragbares Gerät nach Anspruch 10, wobei der Prozessor (142) ferner dazu konfiguriert ist,
vom Benutzer eine Anweisung zum Einstellen eines Betriebsmodus des tragbaren Geräts auf den Trainingsmodus zu empfangen und
den auf das erste Gelenk anzuwendenden Widerstandspegel zu bestimmen.

14. Tragbares Gerät nach Anspruch 10, wobei der Prozessor (142) ferner dazu konfiguriert ist,
vom Benutzer eine Anweisung zum Einstellen eines Betriebsmodus des tragbaren Geräts auf einen Unterstützungsmodus zu empfangen;
auf der Grundlage eines ersten Winkels einen Unterstützungsdrehmomentwert zu berechnen, der im Unterstützungsmodus auf das erste Gelenk anzuwenden ist; und
dem Benutzer eine Unterstützungskraft durch Steuern des Motors auf der Grundlage des Unterstützungsdrehmomentwerts bereitzustellen.

## Revendications

1. Procédé de commande d'un circuit d'entraînement de moteur (112 ; 1400 ; 1800 ; 1900) d'un équipement portable (100), le procédé comprenant :
commander un premier commutateur (710 ; 1410 ; 1810 ; 1950) et un deuxième commutateur (720 ; 1430 ; 1830 ; 1930) d'un circuit d'entraînement de moteur (112 ; 1400 ; 1800 ; 1900) de sorte qu'une batterie (150) ne forme pas une boucle fermée passant à travers le circuit d'entraînement de moteur (112 ; 1400 ; 1800 ; 1900) en réponse à ce qu'un mode d'exercice soit défini dans l'équipement portable (100) ;
déterminer (1140 ; 1340) un niveau de résistance à appliquer à une première articulation d'un utilisateur ;
déterminer (1150 ; 1350) un rapport de connexion entre une durée de connexion pendant laquelle des bornes d'un moteur (114) doivent être connectées électriquement en boucle fermée et une durée de déconnexion pendant laquelle les bornes du moteur doivent être déconnectées électriquement, en fonction du niveau de résistance ; et
commander (1160 ; 1360) un troisième commutateur (730 ; 1420 ; 1820 ; 1910) et un quatrième commutateur (740 ; 1440 ; 1840 ; 1940) du circuit d'entraînement de moteur (112 ; 1400 ; 1800 ; 1900) connecté électriquement au moteur (114) en fonction du rapport de connexion.

2. Procédé selon la revendication 1, dans lequel le rapport de connexion est représenté par une modulation de largeur d'impulsion, PWM.

3. Procédé selon la revendication 2, dans lequel un niveau de résistance à fournir à l'utilisateur est réglé en fonction du rapport de connexion de sorte que, lorsque la durée de connexion pendant laquelle les bornes du moteur (114) doivent être connectées électriquement en boucle fermée augmente, la force de résistance augmente.

4. Procédé selon la revendication 1, dans lequel, dans un état dans lequel les bornes du moteur (114) doivent être connectées électriquement en boucle fermée, le moteur (114) est configuré pour fonctionner comme un générateur par rapport à une force externe exercée par l'utilisateur.

5. Procédé selon la revendication 4, comprenant en outre :
recharger la batterie (150) de l'équipement portable en utilisant de l'énergie générée par le générateur, lorsque le moteur (114) fonctionne comme le générateur.

6. Procédé selon la revendication 1, comprenant en outre :
recevoir, à partir de l'utilisateur, une instruction pour définir un mode de fonctionnement de l'équipement portable en mode d'exercice.

7. Procédé selon la revendication 6, dans lequel, lorsque le mode d'exercice est défini, le moteur (114) n'est pas fourni d'énergie de la batterie (150) de l'équipement portable.

8. Procédé selon la revendication 1, comprenant en outre :
recevoir, à partir de l'utilisateur, une instruction pour définir un mode de fonctionnement de l'équipement portable en mode d'assistance ;
calculer une valeur de couple d'assistance à appliquer à la première articulation en mode assistance en fonction d'un premier angle ; et
fournir une force d'assistance à l'utilisateur en commandant le moteur en fonction de la valeur de couple d'assistance.

9. Support non transitoire lisible par ordinateur comprenant des instructions lisibles par ordinateur pour amener un ordinateur à mettre en œuvre le procédé selon la revendication 1 sur un équipement portable comprenant un moteur (114), un circuit d'entraînement de moteur (112 ; 1400 ; 1800 ; 1900), un premier commutateur (710 ; 1410 ; 1810 ; 1950), un deuxième commutateur (720 ; 1430 ; 1830 ; 1930), un troisième commutateur (730 ; 1420 ; 1820 ; 1910) un quatrième commutateur (740 ; 1440 ; 1840 ; 1940) et un processeur est configuré pour mettre en œuvre le procédé selon la revendication 1.

10. Équipement portable (100) configuré pour commander un circuit d'entraînement de moteur (112 ; 1400 ; 1800 ; 1900) de l'équipement portable (100), l'équipement portable (100) comprenant :
une mémoire (144) configurée pour stocker un programme comprenant des instructions destinées à fournir la force de résistance à l'utilisateur ;
un circuit d'entraînement de moteur (112 ; 1400 ; 1800 ; 1900) ;
un moteur (114) connecté électriquement au circuit d'entraînement de moteur (112 ; 1400 ; 1800 ; 1900) ; et
un processeur (142) configuré pour exécuter le programme pour
commander un premier commutateur (710 ; 1410 ; 1810 ; 1950) et un deuxième commutateur (720 ; 1430 ; 1830 ; 1930) du circuit d'entraînement de moteur (112 ; 1400 ; 1800 ; 1900) de sorte qu'une batterie (150) ne forme pas une boucle fermée passant à travers le circuit d'entraînement de moteur (112 ; 1400 ; 1800 ; 1900) en réponse à ce qu'un mode d'exercice soit défini dans l'équipement portable (100),
déterminer (1140 ; 1340) un niveau de résistance à appliquer à une première articulation de l'utilisateur,
déterminer (1150 ; 1350) un rapport de connexion entre une durée de connexion pendant laquelle des bornes d'un moteur (114) doivent être connectées électriquement en boucle fermée et une durée de déconnexion pendant laquelle les bornes du moteur doivent être déconnectées électriquement, en fonction du niveau de résistance, et
commander (1160 ; 1360) un troisième commutateur (730 ; 1420 ; 1820 ; 1910) et un quatrième commutateur (740 ; 1440 ; 1840 ; 1940) du circuit d'entraînement de moteur (112 ; 1400 ; 1800 ; 1900) connecté électriquement au moteur (114) en fonction du rapport de connexion.

11. Équipement portable selon la revendication 10, dans lequel le rapport de connexion est représenté par une modulation de largeur d'impulsion, PWM, et
une force de résistance à fournir à l'utilisateur est réglé en fonction du rapport de connexion de sorte que, lorsque la durée de connexion pendant laquelle les bornes du moteur (114) doivent être connectées électriquement en boucle fermée augmente, la force de résistance augmente.

12. Équipement portable selon la revendication 10, dans lequel, dans un état dans lequel les bornes du moteur (114) doivent être connectées électriquement en boucle fermée, le moteur (114) est configuré pour fonctionner comme un générateur par rapport à une force externe exercée par l'utilisateur.

13. Équipement portable selon la revendication 10, dans lequel le processeur (142) est configuré en outre pour
recevoir, à partir de l'utilisateur, une instruction pour définir un mode de fonctionnement de l'équipement portable en mode d'exercice, et
déterminer le niveau de résistance à appliquer à la première articulation.

14. Équipement portable selon la revendication 10, dans lequel le processeur (142) est configuré en outre pour
recevoir, à partir de l'utilisateur, une instruction pour définir un mode de fonctionnement de l'équipement portable en mode d'assistance ;
calculer une valeur de couple d'assistance à appliquer à la première articulation en mode assistance en fonction d'un premier angle ; et
fournir une force d'assistance à l'utilisateur en commandant le moteur en fonction de la valeur de couple d'assistance.
